# EUROPÄISCHE PATENTSCHRIFT

(11) **EP 3 996 893 B1**
(45) Veröffentlichungstag und Bekanntmachung des Hinweises auf die Patenterteilung: **08.07.2026**
(21) Anmeldenummer: 20768274.1
(22) Anmeldetag: 08.07.2020
(51) Int. Cl.: B29C 39/08, B29C 39/10, B29C 39/24, B29C 33/00, B29C 33/12, B29C 33/30

(54) **VERGUSSVORRICHTUNG FÜR EINEN GASTAUSCHER**
POTTING DEVICE FOR A GAS EXCHANGER
DISPOSITIF D'ENROBAGE POUR UN ÉCHANGEUR DE GAZ

(30) Priorität: 11.07.2019 DE 102019004887
(43) Veröffentlichungstag der Anmeldung: 18.05.2022
(73) Patentinhaber: Xenios AG, 74076 Heilbronn (DE)
(72) Erfinder: RAU, Michael, 74177 Bad Friedrichshall (DE); SCHRAVEN, Lotte, 52072 Aachen (DE); FILPON, Sven, 74080 Heilbronn (DE); ROSSBROICH, Ralf, 80469 München (DE)
(74) Vertreter: Graf von Stosch Patentanwaltsgesellschaft mbH
(86) Internationale Anmeldenummer: PCT/EP2020/069309
(87) Internationale Veröffentlichungsnummer: WO 2021/005132

(56) Entgegenhaltungen:
- EP-A2- 0 138 763
- EP-B1- 0 138 763
- WO-A1-03/051495
- WO-A2-03/006134
- DE-A1- 102006 021 066
- DE-A1- 102010 033 826
- DE-B3- 102018 209 444
- GB-A- 1 590 045
- JP-A- H0 819 729
- JP-A- S51 103 083
- KR-A- 20050 093 885
- US-A- 3 923 209
- US-A1- 2005 115 885
- US-B2- 10 286 137

## Beschreibung

### Technisches Gebiet

Die vorliegende Erfindung betrifft eine Vergussvorrichtung zum Herstellen eines Vergusses für einen Gastauscher sowie ein Verfahren zur Herstellung eines solchen Vergusses.

### Stand der Technik

In externen Lungensystemen wird Blut von einem Patienten über einen Patientenzugang entnommen und über eine Gasaustauschmembran geführt, um somit eine CO₂-Entreicherung und Oxygenierung des Blutes zu bewirken, wenn der physiologische Zustand des Patienten dies nicht hinreichend unterstützt. Um eine eventuelle Pumpenunterstützung zu vermeiden, kann das Blut von einem arteriellen Patientenzugang entnommen werden, so dass der patienteneigene Kreislauf die Entnahme und Förderung des Blutes durch das Lungensystem bewirkt. Dies erfordert jedoch, dass der Widerstand im Lungensystem bzw. der Gasaustauschmembran möglichst gering gehalten wird und das Lungensystem relativ klein bemessen ist.

Entsprechend können Lungensysteme eine Vielzahl an Gasaustauschmembranen umfassen, welche entweder aufeinandergestapelt oder ineinander gewickelt sind, beispielsweise als Membranmatten, welche eine Vielzahl von semipermeablen Hohlfasern aufweisen und einen Gasaustausch, aber keine Übertragung von Flüssigkeiten ermöglichen. Auf diese Weise kann Blut von einer Anschlussseite des Systems über die Membran geführt und anschließend über einen stromabwärtsgelegenen Anschluss dem Patienten wieder zurückgeführt werden, wobei durch die Hohlfasern der Membran Sauerstoff geführt wird. Durch die hohe Affinität des Hämoglobins an Sauerstoff wird über den Diffusionsgradienten eine Oxygenierung des Blutes bei einer gleichzeitigen Entreicherung des im Blut gelösten Kohlenstoffdioxids bewirkt.

Durch die mattenförmige Struktur der Membran wird weiterhin die Gasaustauschoberfläche maximiert. Dies erfordert jedoch gleichzeitig, dass das einströmende und ausströmende Blut sowie die Hohlfasern hinreichend abgedichtet sein müssen, um eine Verschmutzung des Innenraums des Gastauschers zu verhindern und die Sicherheit des Patienten nicht zu gefährden. Diese Abdichtung wird herkömmlich mittels eines Vergusses bewirkt, wobei Vergussmaterial in einen Trichter eingegeben und über den Gastauscher durch die umgebende Kassette verteilt wird, um den Gastauscher unter Einwirkung von Zentrifugalkräften an den Umfangsflächen abzudichten.

Die Verwendung von herkömmlichen Trichtern hat jedoch den Nachteil, dass die Verteilungskanäle nach Verwendung wegen des ausgehärteten Vergussmaterials verstopft sind und entfernt werden kann. Die Trichter können daher nur als Einmalartikel verwendet werden. Dadurch geht die Herstellung des Vergusses nicht nur mit einer hohen Abfalllast, sondern auch mit hohen Kosten einher, beispielsweise im Falle einer 3D-Druck-Herstellung des Einmaltrichters. Ebenfalls können bei der Herstellung von solchen Trichtern auch Toleranzschwankungen auftreten, so dass die Herstellung des Vergusses ggf. nicht exakt reproduzierbar ist.

Weiterhin ist der Reinigungsprozess der Kassetten aufwendig. Es können Undichtigkeiten zwischen der Kassette und den darin enthaltenen Gastauscher-Elementen auftreten, beispielsweise zwischen der Kassette und einem Verschluss oder Endkappe, was eine erhöhte Nacharbeit erfordert und eine Verschmutzung des Reinraums bewirkt. Diese Undichtigkeiten werden durch den langsamen und ungleichmäßigen Temperaturausgleich der Kassetten verstärkt. Dies gilt umso mehr, als nicht nur die Prozesszeiten aufgrund der langen Aufheiz- und Abkühlzeiten beeinflusst werden, sondern auch ein erhöhter Schwund des Vergussmaterials auftreten kann. Beispielsweise können durch längere Abkühlzeiten und die ungleichmäßige Abkühlung Schwund und Blasenbildung im Vergussmaterial auftreten, welche die strukturellen Eigenschaften und auch die Abdichtung der Gastauscher-Elementen beeinträchtigen. Eventuelle Rückstände des Vergussmaterials können somit zusammen mit den prozessbedingten Undichtigkeiten die Wirkung des Vergusses reduzieren und die Sicherheit des Patienten potenziell gefährden.

Zusätzlich zu den unvorteilhaften thermischen Eigenschaften wird die Qualität bzw. die strukturelle Stabilität von verschiedenen Faktoren, wie die Vergussmaterialmenge und die Vergusszeit bzw. die Steuerung, sowie von Toleranzschwankungen der Gastauscher-Elemente beeinflusst, die abhängig von den gewählten Elementen individuell unterschiedlich sein können. Beispielsweise können starke Schwankungen der einzusetzenden Vergussmaßen bestehen, welche aufgrund von Materialtoleranzen und des gewünschten definierten Durchmessers entstehen. Dabei ist die Verteilung des Vergussmaterials oft nicht gleichmäßig, so dass die Vergussvorrichtung mehrmals erneut auf die Zentrifuge aufgespannt werden muss. Hierdurch werden die bereits langen Prozesszeiten durch den Umspannprozess und die zusätzlichen Vergusszeiten weiter erhöht. Entsprechend wird der Verguss mehrstufig hergestellt, wodurch Bindenähte entstehen, die das Risiko von Undichtigkeiten erhöhen.

Daher ist es schwierig, eine Herstellung eines Vergusses mit einem reproduzierbaren Durchmesser zu ermöglichen bzw. sicherzustellen.

US 10,286,137 B2 beschreibt eine Vergussvorrichtung mit einem Verteiler und einer Kassette. US 3,923,209 A beschreibt einen Verteiler, bei dem eine Anzahl sektorförmiger Elemente zwischen einem Nabenelement und einem Klemmring angeordnet sind.

Entsprechend besteht ein Bedarf, die Herstellung eines Vergusses für einen Gastauscher derart zu optimieren, dass eine höhere strukturelle Integrität des Vergusses bei einer vereinfachten und kürzeren Herstellung bereitgestellt wird.

### Darstellung der Erfindung

Ausgehend von dem bekannten Stand der Technik ist es eine Aufgabe der vorliegenden Erfindung, eine verbesserte Herstellung eines Vergusses für einen Gastauscher zu ermöglichen.

Die Aufgabe wird durch die unabhängigen Ansprüche gelöst. Vorteilhafte Weiterbildungen ergeben sich aus den Unteransprüchen, der Beschreibung und den Figuren.

Entsprechend wird eine Vergussvorrichtung zum Herstellen eines Vergusses für einen Gastauscher unter Einwirkung einer Zentrifugalkraft vorgeschlagen. Die Vergussvorrichtung umfasst einen Verteiler, welcher eine Öffnung und mindestens einen durchgehenden Kanal umfasst und dazu ausgelegt ist, ein fluidisches Vergussmaterial über die Öffnung zu empfangen und über den mindestens einen Kanal zu führen. Weiterhin umfasst die Vergussvorrichtung eine Kassette, welche einen inneren Hohlraum zum Aufnehmen von Gastauscher-Elementen definiert, welcher fluidisch mit dem mindestens einen Kanal verbunden ist. Der Verteiler umfasst mindestens zwei Verteilerkomponenten, welche im zusammengesetzten Zustand des Verteilers die Öffnung definieren, auslaufsicher miteinander verbunden sind und zwischen aneinandergrenzenden Bereichen den mindestens einen Kanal bilden. Weiterhin umfasst die Vergussvorrichtung (10) eine Formdichtung (44), wobei die Kassette (16) verschließbar und zum Aufnehmen der Formdichtung (44) ausgebildet ist, wobei die Formdichtung (44) den inneren Hohlraum (46) vollständig umschließt und diesen bis zum mindestens einen Kanal (28) fluidsicher abdichtet.

Der Verteiler kann beispielsweise auf eine Oberseite der Kassette aufgesetzt und daran befestigt werden, beispielsweise mittels Schrauben oder Klemmen, entweder formschlüssig oder kraftschlüssig. Dabei können die Verteilerkomponenten zunächst zusammengesetzt oder zusammengesteckt werden oder sie können nacheinander auf die Kassette aufgesetzt werden, um derart den Verteiler auf der Kassette zu bilden. Obwohl die Anordnung der Verteilerkomponenten bereits deren Verbindung bewirkt, können diese weiterhin miteinander mittels einer Fixiervorrichtung verbunden werden, beispielsweise mittels eines Klemmrings, der stufenweise oder stufenlos festgezogen oder festgedreht werden kann. Hierdurch kann eine verbesserte Abdichtung zwischen den jeweiligen Verteilerkomponenten erreicht werden.

Eine Anordnung an der Oberseite hat den Vorteil, dass das Eingeben des Vergussmaterials von oben erfolgen kann, beispielsweise über eine Düse, die in die Öffnung eingeführt wird. Dies kann insbesondere vorteilhaft für Vergussmaterialien sein, die sich aufgrund ihrer Materialeigenschaften, wie Viskosität oder Benetzungseigenschaften, nicht gut verteilen lassen und somit - von Schwerkraft unterstützt - in den Kanal fließen können.

Der Kanal ermöglicht weiterhin einen Fluss des Vergussmaterials von der Öffnung zum inneren Hohlraum der Kassette und stellt mithin eine Fluidverbindung bereit. Der Kanal ist zwischen aneinandergrenzenden Bereichen der Verteilerkomponenten ausgebildet. Mit anderen Worten wird durch eine entsprechende Formgebung der Verteilerkomponenten an einer Grenzfläche, beispielsweise eine halbkreisförmige Durchbohrung, und die entsprechende Anordnung der Verteilerkomponenten, wo die Grenzflächen einander berühren, ein Kanal gebildet. So können beide Grenzflächen mit einer halbkreisförmigen Durchbohrung versehen sein, so dass beim Zusammensetzen des Verteilers ein kreisförmiger Kanal gebildet wird.

Alternativ kann jedoch auch an nur einer Grenzfläche eine halbkreisförmige Durchbohrung vorgesehen sein, so dass die halbkreisförmige Durchbohrung beim Zusammensetzen des Verteilers von einer geraden Fläche begrenzt wird und der Kanal entsprechend sich halbkreisförmig oder als Halbzylinder erstreckt. Die entsprechende Grenzfläche kann den mindestens einen Kanal bilden. Statt einer Halbkreisform können alternativ andere Formen, wie eine rechteckige oder ellipsoide Form, vorgesehen sein.

Durch die Anordnung des Kanals an der Grenzfläche bzw. zwischen den Verteilerkomponenten wird eine Entfernung des Vergussmaterials erleichtert, indem der Kanal geöffnet und vollständig gereinigt werden kann. Während dies in herkömmlichen Trichtern, die aus einem Bauteil bestehen oder einen internen Kanal umfassen, aufgrund der Festigkeit nach dem Aushärten der Vergussmasse nicht möglich ist, ermöglicht die Unter- bzw. Aufteilung des Verteilers und die Anordnung des Kanals eine einfache Loslösung der Vergussmasse bzw. des Vergussmaterials. Eine solche Aufteilung kann beispielsweise durch Lösen eines Klemmrings und Lösen von Fixierschrauben erreicht werden, wonach die Verteilerkomponenten anschließend nach außen von der Kassette weggezogen werden können.

Die Vergussvorrichtung und insbesondere die Verteilerkomponenten können somit leicht entformt werden, so dass die Komponenten der Vorrichtung leicht entnommen und gereinigt werden können, ohne größere Rückstände des Vergussmaterials zu hinterlassen. Die Verteilerkomponenten können daher ohne Hinterschnitte ausgebildet sein und beispielsweise Entformschrägen aufweisen oder teilweise abgerundet sein. Es handelt sich bei den Verteilerkomponenten also vorteilhafterweise um Komponenten, welche lösbar miteinander verbunden werden können, sodass das Zusammensetzen des Verteilers reversibel ist.

Optional kann weiterhin vorgesehen sein, dass die Verteilerkomponenten an dem Bereich, der mit der Kassette verbunden wird, eine oder mehrere Aussparungen aufweisen, beispielsweise zwei keilförmige Aussparungen, so dass das Einführen eines entsprechenden keilförmigen Elements und/oder einer Gabel eine Presspassung und/oder eine Hebelkraft bewirkt. Dies erleichtert die Abtrennung der jeweiligen Verteilerkomponente von der Kassette, während das ausgehärtete Vergussmaterial zurückbleibt. Somit kann der Kanal, der einen Zulauf für den inneren Hohlraum der Kassette bildet, freigelegt werden und die Abtrennung von der Kassette bereits in einer früheren Phase erfolgen.

Der Kanal bzw. der Zulauf ist weiterhin bevorzugt mittels einer Dichtung mit dem inneren Hohlraum fluidisch verbunden, so dass kein Vergussmaterial zwischen dem Kanal und der Kassette austreten kann. Beispielsweise kann der Kanal in Umfangsrichtung bzw. umlaufend von der Dichtung umgeben sein, so dass die Dichtung als Flachdichtung oder O-Ring ausgebildet sein kann. Die Kassette kann weiterhin für jeden Kanal eine Bohrung aufweisen, welche den Kanal mit dem inneren Hohlraum fluidisch verbindet, so dass das Vergussmaterial durch die Bohrung über einen Vergussraum verteilt werden kann.

Im inneren Hohlraum können verschiedene Gastauscher-Elemente vorgesehen sein. Beispielsweise können an gegenüberliegenden Abschnitten der Kassette, beispielsweise an einer in Richtung der Zentrifugalachse gesehenen Oberseite und Unterseite, Verschlüsse und dazwischen angeordnete Gastauschmembranen in Form von Hohlfasermatten angeordnet sein. Der Kanal kann dabei so angeordnet sein, dass das Einführen des Vergussmaterials einen Verguss an den abzudichtenden Abschnitten des Gastauschers bereitstellt. Dazu kann die Vergussvorrichtung auf einer Zentrifuge montiert werden, beispielsweise über eine entsprechende Konfiguration des unteren Abschnitts der Kassette.

Während der Herstellung kann das Vergussmaterial dann über eine Düse in die Öffnung eingeführt und über den Kanal in den inneren Hohlraum gebracht werden, wobei die Vergussmenge und die Form der Kassette die Dicke bzw. Stärke des Vergusses vorgeben können. Durch Aushärten des Vergussmaterials während des Zentrifugierens wird ein Verguss hergestellt, welcher die Gastauscher-Elemente abschließt. Das Aushärten kann bereits bei Umgebungstemperatur erfolgen. Optional kann zum Aushärten des Vergussmaterials jedoch auch eine Erhöhung der Temperatur auf eine bevorzugte Reaktionstemperatur vorgesehen sein, um das Vergussbild weiter zu optimieren.

Das Vergussmaterial kann je nach gewünschten Eigenschaften des Vergusses gewählt werden, so dass der Gastauscher beispielsweise eine vorgegebene Festigkeit und/oder Transparenz aufweisen kann. Beispielsweise kann das Vergussmaterial Polyurethan sein und als Kunstharz in die Öffnung eingeführt werden. Als Vergussmaterial können jedoch grundsätzlich jegliche Materialien gewählt werden, welche bei Umgebungstemperatur zunächst flüssig und zum Vermischen geeignet sind und erst nach einer vorgegebenen Zeit beginnen auszuhärten. Beispielsweise kann das Vergussmaterial alternativ Silikon umfassen oder daraus bestehen.

Bevorzugt bilden die Verteilerteile im zusammengesetzten Zustand des Verteilers mindestens zwei durchgehende Kanäle. Dadurch können unterschiedliche Bereiche des inneren Hohlraums gleichzeitig über die zentrale Öffnung befüllt werden, so dass die Verteilung des Vergussmaterials optimiert werden kann. So können die Kanäle an - in einer radialen Richtung zur Zentrifugalachse sich erstreckenden - gegenüberliegenden Bereichen der Kassette angeordnet sein. Der innere Hohlraum und eventuell vorhandene Bohrungen der Kassette oder Eingangsbereiche des Hohlraums können derart geformt sein, dass das durch jeden Kanal eingespeiste Vergussmaterial die Gastauscher-Elemente halbkreisförmig umschließt. Dadurch können die Gastauscher-Elemente vollständig vom Vergussmaterial umschlossen werden, indem das Vergussmaterial eine äußere Hülle des Gastauschers bilden kann.

Die Verteilerkomponenten können ebenfalls mehr als zwei Kanäle bilden. Beispielsweise können drei Kanäle gebildet werden, wobei ein dritter Kanal zwischen zwei an gegenüberliegenden Abschnitten der Kassette angeordneten Kanälen angeordnet ist, um einen Zwischenraum des Gastauschers mit einem Verguss zu versehen und/oder eine weitergehende strukturelle Stabilität zu bewirken. Es können ebenfalls vier Kanäle vorgesehen sein, wobei an den gegenüberliegenden Abschnitten der Kassette jeweils zwei Kanäle angeordnet sind, die derart voneinander beabstandet sind, dass sie das Vergussmaterial in unterschiedlichen Bereichen des inneren Hohlraums einspeisen, beispielsweise durch eine entsprechende Formgebung des inneren Hohlraums und/oder einer vorhandenen Bohrung der Kassette.

Die Mehrzahl der Kanäle kann ebenfalls von mehr als zwei Verteilerkomponenten ausgebildet sein. So können beispielsweise drei Verteilerkomponenten vorgesehen sein, welche im zusammengesetzten Zustand einen kreisförmigen bzw. zylinderförmigen oder rechteckigen Verteiler bilden und an drei Grenzflächen jeweils einen Kanal definieren. Entsprechend können die Anzahl der Kanäle und Verteilerkomponenten sowie die Anordnung der Kanäle an die Einzelheiten und Eigenschaften des auszubildenden Gastauschers angepasst werden.

Um das Zusammensetzen des Verteilers und die Anordnung bzw. die Befestigung des Verteilers auf der Kassette zu erleichtern, ist der Verteiler weiterhin bevorzugt aus gleichförmigen Verteilerkomponenten gebildet. Beispielsweise kann der Verteiler im Wesentlichen zylinderförmig ausgebildet sein, wobei jede Verteilerkomponente einen entsprechenden Abschnitt des Zylinders bildet, beispielsweise ein Halbkreis, wenn zwei Verteilerkomponenten vorgesehen sind, oder einen Viertelkreis, wenn vier Verteilerkomponenten vorgesehen sind. Entsprechend können die Verteilerkomponenten auch Teile eines Rechtecks bilden.

Die Verteilerkomponenten sind dabei bevorzugt symmetrisch ausgebildet, so dass das Zusammensetzen keiner bestimmten Ausrichtung der jeweiligen Verteilerkomponente bedarf, sondern lediglich erfordert, dass die Grenzflächen aneinandergrenzen. Hierdurch wird die Herstellung der Verteilerkomponenten erheblich erleichtert, da nur eine Form bzw. ein Typ der Verteilerkomponente hergestellt werden muss, um die avisierte Form des Verteilers zu ermöglichen.

Obwohl generell auch eine Aneinanderreihung der Verteilerkomponenten möglich ist, grenzt jede Verteilerkomponente im zusammengesetzten Zustand des Verteilers bevorzugt an zwei Verteilerkomponenten an und bildet mit der angrenzenden Verteilerkomponente einen Kanal. Daher sind mindestens drei Verteilerkomponenten vorgesehen, bevorzugt vier, wobei jede Verteilerkomponente an gegenüberliegenden Grenzflächen einen Teil eines Kanals definiert, der im zusammengesetzten Zustand des Verteilers mit der Grenzfläche der jeweils angrenzenden Verteilerkomponente einen Kanal bildet. Entsprechend kann der Verteiler mindestens drei oder mindestens vier, vorzugsweise drei bis fünf oder, weiter vorzugsweise drei oder vier Kanäle umfassen. Bevorzugt bildet jede Verteilerkomponente dabei einen Viertelkreis oder einen Winkel, wobei die Grenzflächen an den Stirnseiten vorgesehen sind. Mit anderen Worten werden die Kanäle bevorzugt in den Bereichen gebildet, wo die Verteilerkomponenten miteinander verbunden werden, um den Kreis bzw. das Rechteck auszubilden.

Da Gastauscher in der Regel eine Abdichtung von mehreren Seiten erfordern, ist der Verteiler weiter bevorzugt aus drei oder vier gleichförmigen Verteilerkomponenten gebildet. Dadurch können vier Kanäle bereitgestellt werden, welche in Umlaufrichtung gleichmäßig voneinander beabstandet sind und jeweils eine Seite des auszubildenden bzw. des abzudichtenden Gastauschers mit dem Vergussmaterial versehen.

Entsprechend kann an den Seiten der Verteilerkomponenten jeweils eine Hälfte eines Kanals vorgesehen sein, so dass beim Zusammenstellen der Verteilerkomponenten insgesamt vier vollständige Kanäle entstehen, welche eine gleichmäßige Verteilung des Vergussmaterials ermöglichen. Weiterhin werden dadurch die Fließwege des Vergussmaterials erheblich verkürzt, so dass das Abdichten effizienter und effektiver erfolgen kann und die Vergussvorrichtung entsprechend nur ein einziges Mal auf der Zentrifuge montiert werden muss bzw. das Herstellungsverfahren ununterbrochen erfolgen und somit verkürzt werden kann. Dadurch werden ebenfalls Bindernähte vermieden, so dass das Risiko von Undichtigkeiten des Gastauschers reduziert wird.

In Abhängigkeit von den Prozessbedingungen können die Verteilerkomponenten aus einem Material gebildet sein, das eine auslaufsichere Abdichtung zwischen den Verteilerkomponenten im zusammengesetzten Zustand des Verteilers bewirkt, beispielsweise bei partieller Verwendung eines Elastomers. Alternativ können weitere Dichtungsmittel vorgesehen sein. Im Ergebnis kann jede Verteilerkomponente eine innere Dichtung und eine äußere Dichtung an gegenüberliegenden Seiten des jeweiligen Kanals umfassen, welche jeweils den Kanal im zusammengesetzten Zustand des Verteilers fluidisch abdichten.

Eine solche Dichtung kann beispielsweise in Form einer Schnur bereitgestellt sein, so dass die Verteilerkomponenten zueinander mit je zwei Dichtschnüren abgedichtet sind und so einen Austritt des Vergussmaterials während laufender Zentrifuge in die Umgebung verhindern. Die Dichtschnur kann auf einer Seite des Verteilerteils bzw. an einer Grenzfläche in eine an der Grenzfläche vorgesehene Dichtnut eingelegt werden, wobei die gegenüberliegende Grenzfläche der jeweils anderen Verteilerkomponente glatt ist und somit eine optimale Dichtfläche bereitstellt. Die Dichtschnüre erstrecken sich bevorzugt ausgehend von der Öffnung bis zur Kassette bzw. einer Bohrung der Kassette, wobei die Kassette an dieser Stelle ebenfalls eine Dichtung in Form einer Flachdichtung aufweisen kann, wie vorstehend beschrieben. Somit wird sichergestellt, dass das in die Öffnung eingeführte Vergussmaterial über den Kanal in den inneren Hohlraum der Kassette fließen kann, ohne wegen der Zentrifugalkräfte aus der Vergussvorrichtung auszutreten.

Zum Eingeben bzw. Einspeisen des Vergussmaterials kann eine Düse oder eine andere Füllvorrichtung vorgesehen sein, welche in der Öffnung angeordnet ist. Um das Eingeben zu erleichtern und das Eingeben über die Öffnung im Wesentlichen unabhängig von der spezifischen Ausgestaltung der Füllvorrichtung zu ermöglichen, definiert die Öffnung bevorzugt einen trichterförmigen Füllbereich für das Vergussmaterial. Somit können verschiedene Flussgeschwindigkeiten des Vergussmaterials - ohne Anpassung des Verteilers - und unterschiedliche Vergussmaterialien zur Herstellung des Vergusses gewählt werden. Durch die Trichterform wird nämlich eine - im Verhältnis zur Öffnung der Füllvorrichtung - größere Oberfläche bereitgestellt, so dass eventuell abprallendes Vergussmaterial abgefangen und in den jeweiligen Kanal geführt wird. Mit anderen Worten ist die Trichterform der Öffnung so zu verstehen, dass die Öffnung einen trichterförmigen Öffnungsbereich stromaufwärts des jeweiligen Kanals bereitstellt.

Wegen eventueller Abweichungen der Prozessbedingungen kann die erforderliche Menge des Vergussmaterials variieren. Um ein genaues Abmessen des Vergussmaterials zu vermeiden und diese Abweichungen auszugleichen, definieren die Verteilerkomponenten bevorzugt weiterhin im zusammengesetzten Zustand des Verteilers einen Überlauf, welcher derart angeordnet und geformt ist, dass ein Materialfluss über eine vorgegebene Grenzfläche im inneren Hohlraum einen Materialfluss in den Überlauf bewirkt.

Der Überlauf kann an die Öffnung bzw. den Füllbereich angepasst sein bzw. zusammen mit diesem Bereich eine Führung des überschüssigen Vergussmaterials bewirken. Beispielsweise kann der Überlauf so geformt und angeordnet sein, dass das überschüssige Vergussmaterial in den Überlauf läuft und dort gesammelt wird, wenn das Vergussmaterial in dem inneren Hohlraum der Kassette so weit gefüllt ist, dass dies in radialer Richtung den zur Öffnung ausgerichteten Rand des Füllbereichs erreicht.

Der Überlauf und der Füllbereich haben demnach einen auf den inneren Hohlraum und/oder den darin enthaltenen Gastauscher präzise abgestimmten Durchmesser, so dass das Eingeben des Vergussmaterials weder über die Menge noch über die Zeit gesteuert werden müssen und Abweichungen in Hinblick auf Zeit, Vergussmaterial und Abmessungen der Gastauscher-Elemente eliminiert werden können.

Durch den Überlauf wird auch ermöglicht, dass das Herstellungsverfahren kontinuierlich ohne Unterbrechung fortlaufen kann, so dass Bindernähte vermieden und Prozesszeiten verkürzt werden können.

Um Temperaturschwankungen weitestgehend zu vermeiden und eine verbesserte thermische Verteilung innerhalb der Kassette zu ermöglichen, verläuft die Öffnung bevorzugt bis zu einer äußeren Oberfläche der Kassette und verbindet diese fluidisch mit der Umgebung. Die Öffnung ist bevorzugt konzentrisch mit einer Zentrifugalachse der Vergussvorrichtung.

Die Kassette ist in herkömmlichen Vergussvorrichtungen an einer Oberseite oft von einem Verteiler oder Trichter eingeschlossen und an einer Unterseite zumindest teilweise mit einem Zentrifugen-Element gekoppelt. Hierdurch können Temperaturschwankungen auftreten, und es kann nicht nur eine verlängerte Abkühlzeit erforderlich sein, sondern die Abkühlung ihrerseits ungleichmäßig sein. Demzufolge können Schwund und Blasenbildung auftreten, wodurch das Risiko von Undichtigkeiten vergrößert wird.

Durch die Erstreckung der Öffnung bis zur äußeren Oberfläche der Kassette wird jedoch ein thermischer Austausch zwischen der Kassette und der Umgebung ermöglicht, so dass die Umgebungstemperatur eine Belüftung bereitstellt und die Herstellung des Vergusses unterstützen kann. Die konzentrische Anordnung hat auch den Vorteil, dass eine größere zentrale Fläche erreicht und eine verbesserte thermische Ableitung ermöglicht wird.

Daher kann eine Herstellung mit verhältnismäßig kurzen Aufheiz- und Abkühlzeiten und/oder mit kurzen Prozesszeiten erreicht werden. Durch die verkürzten Abkühlzeiten kann der Schwund erheblich reduziert werden. Weiterhin kann dies durch die Umgebungstemperatur auch aktiv geregelt werden, beispielsweise durch eine gezielte Regelung der Umgebungstemperatur. So kann beispielsweise eine Umgebungstemperatur zwischen etwa 40°C und 70°C für Gastauscherfasern bzw. eine Gastauschmembran vorteilhaft sein. Hierdurch wird vermieden, dass sichtbaren Schwundeffekte an den Grenzflächen hervorgerufen und die Hohlfasern beschädigt werden. Weiterhin kann eine solche verbesserte Temperaturverteilung und Temperaturregelung insbesondere für runde Vergüsse die Blasenbildung reduzieren.

Auch durch die optimierte Kühlung wird ermöglicht, dass das Herstellungsverfahren kontinuierlich ohne Unterbrechung ausgeführt werden kann, so dass Bindernähte vermieden und Prozesszeiten verkürzt werden können und die strukturelle Stabilität oder Integrität des Vergusses verbessert wird.

Die vorteilhafte Temperaturverteilung kann durch das Material der Kassette weiter optimiert werden. Durch die Beschleunigung der Kühlung und den direkten Kontakt mit der Umgebungsluft kann die Kassette aus verschiedenen Materialien ausgebildet werden, welche die Blasenbildung durch thermische Eigenschaften weiter reduzieren. Beispielsweise kann die Kassette aus Aluminium, Stahl, einer Aluminiumlegierung, einer Stahllegierung oder einem ähnlichen Material mit hoher Wärmeleitfähigkeit und geringem Wärmeausdehnungskoeffizienten gefertigt sein.

Um die Herstellungskosten und auch den durch die Herstellung verursachten Abfall weiter zu reduzieren, ist bevorzugt zumindest der Verteiler wiederverwendbar. Dadurch, dass die Verteilerkomponenten nach erfolgter Herstellung eines Vergusses voneinander abgetrennt und gereinigt werden können, d.h., indem das zurückgebliebene und gegebenenfalls ausgehärtete Vergussmaterial abgelöst und aus den jeweiligen Kanälen entfernt werden kann, ist eine erneute Füllung der Kassette nach erneuter Zusammensetzung der Verteilerkomponenten möglich. Hierzu kann der Verteiler aus einem widerstandsfähigen Material mit hinreichender struktureller Festigkeit ausgebildet sein, so dass der Verteiler für eine Vielzahl von Herstellungsvorgängen verwendet werden kann.

Die Öffnung und der mindestens eine Kanal können weiterhin mit einer Antihaftbeschichtung versehen sein. Dadurch kann das Vergussmaterial leicht aus dem jeweiligen Kanal entnommen werden, ohne größere Rückstände zu hinterlassen. Weiterhin können die Verteilerkomponenten auch leichter von der Kassette abgetrennt werden, so dass die Integrität des im inneren Hohlraum der Kassette hergestellten Vergusses nicht beeinträchtigt wird. Beispielsweise kann die Antihaftbeschichtung nicht nur an das Material des Verteilers, sondern auch an das verwendete Vergussmaterial angepasst werden, beispielsweise Polyurethan. Durch die Antihaftbeschichtung kann die Wiederverwendbarkeit des Verteilers noch häufiger sichergestellt werden.

Zusätzlich zur Antihaftbeschichtung kann das Entformen bzw. das Demontieren der Vergussvorrichtung durch Abrundungen und Entformschrägen, und zwar ohne Hinterschnitte, erleichtert werden.

Die Verteilerkomponenten können aus einem Material, umfassend Aluminium, ausgebildet sein. Mit anderen Worten kann das Material Aluminium umfassen, beispielsweise in einer Legierung, oder das Material kann aus Aluminium bestehen. Aluminium hat den Vorteil, dass die Verteilerkomponenten relativ leicht in der Handhabung und dennoch robust sind, wobei eine Verformbarkeit des Materials gleichzeitig das Demontieren und Reinigen erleichtert. Weiterhin ermöglicht die Herstellung aus Aluminium, dass die Verteilerkomponenten passgenau ausgebildet werden können. Hierdurch wird auch eine Herstellung der Verteilerteile mittels 3D-Druck ermöglicht.

Aluminium hat weiterhin den Vorteil, dass dieses Material einen geringen Wärmeausdehnungskoeffizienten aufweist, so dass die Verteilerkomponenten formstabil sind. Weiterhin kann über Aluminium Wärme gut abgeleitet bzw. abgeführt werden, so dass dadurch die Herstellung des Vergusses weiter optimiert wird. Durch das Aluminium wird ein optimierter Temperaturausgleich bewirkt, so dass das Herstellungsverfahren kontinuierlich ohne Unterbrechung geführt werden kann, Bindernähte vermieden und Prozesszeiten verkürzt werden können und die strukturelle Stabilität oder Integrität des Vergusses verbessert wird.

Die Vergussvorrichtung umfasst weiterhin eine Formdichtung, wobei die Kassette verschließbar und zum Aufnehmen der Formdichtung ausgebildet ist, und wobei die Formdichtung den Innenraum vollständig umschließt und diesen bis zum mindestens einen Kanal fluidsicher abdichtet.

Die Kassette kann dann geöffnet werden, so dass die Formdichtung in den inneren Hohlraum der Kassette eingeführt werden bzw. diesen umschließen kann. Mit anderen Worten befinden sich der innere Hohlraum und die darin angeordneten Gastauscher-Elemente in der Formdichtung, wobei die äußere Oberfläche der Formdichtung an die Innenseite der Kassette angepasst ist. Beispielsweise kann die Formdichtung im Inneren die gewünschte Form des Vergusses und außen die Form des Innenraums der Kassette aufweisen, wobei die Formdichtung eine vollständige Abtrennung des Vergussmaterials zur Kassette bewirkt.

Beispielsweise kann die Formdichtung für den jeweiligen Kanal bzw. eine jeweilige Bohrung der Kassette eine Dichtlippe umfassen, die beim Verschließen der Kassette durch die Kassette einklemmt wird und eine vollständige Abdichtung des inneren Hohlraums gewährleistet. Dadurch können eventuelle Undichtigkeiten zwischen der Kassette und den Gastauscher-Elementen während der Herstellung des Vergusses vermieden werden. Dies erfordert keine Nacharbeit oder Nachbearbeitung am vergossenen Gastauscher, so dass eine potentielle Verschmutzung des Reinraums ebenfalls ausgeschlossen werden kann.

Auch ist dadurch eine Reinigung der Kassetten mit einem geringen Aufwand möglich, während das Material der Formdichtung ein einfaches Abziehen vom Vergussmaterial nach dem Aushärten des Vergussmaterials und nach Öffnung der Kassette ermöglicht. Beispielsweise kann die Formdichtung aus TM6MED gefertigt sein. Somit kann die Kassette frühzeitig von den vergossenen Gastauscher-Elementen getrennt werden, wodurch die Herstellungszeiten weiter gekürzt werden können.

Bevorzugt ist die Formdichtung aus zwei symmetrisch ausgebildeten und koppelbaren Formdichtungskomponenten ausgebildet. Dies ermöglicht eine einfache Anordnung und vereinfacht die Herstellung der Formdichtungen. Beispielsweise kann die Formdichtung aus einer oberen und einer unteren Formdichtungskomponente gebildet werden, wobei die obere Formdichtungskomponente eine fluidische Verbindung mit dem Kanal bereitstellt, beispielsweise durch Durchstechen eines entsprechenden Bohrungsanschlusses. Die Gastauscher-Elemente können auf die untere Formdichtungskomponente angeordnet werden, und die obere Formdichtungskomponente kann anschließend einfach aufgesetzt werden, um die Formdichtung abzuschließen.

Um eine noch bessere Temperaturverteilung und Kühlung zu ermöglichen und die Komplexität der Kassette und der Formdichtung zu reduzieren, können diese zumindest teilweise nichtdurchgehende Flächen aufweisen. So kann vorgesehen sein, dass die Kassette aus einem in Richtung einer Zentrifugalachse der Vergussvorrichtung oberen und unteren koppelbaren Kassettenbauteil ausgebildet ist, wobei die Kassettenbauteile zum Aufnehmen einer jeweiligen Formdichtungskomponente ausgebildet sind und wobei die Kassettenbauteile und die Formdichtungskomponente jeweils eine Aussparung oder Öffnung zur Aufnahme eines Gastauscher-Verschlusses umfassen, welche konzentrisch mit der Zentrifugalachse der Vergussvorrichtung ist.

Dies ermöglicht eine einfache Montage, wobei die erste Formdichtungskomponente in das untere Kassettenbauteil und der Gastauscher-Verschluss sowie die Gastauscher-Elemente auf die erste Formdichtungskomponente eingesetzt werden. Danach kann ein gegenüberliegender Gastauscher-Verschluss sowie die zweite Formdichtungskomponente auf der ersten Formdichtungskomponente und das obere Kassettenbauteil auf die zweite Formdichtungskomponente aufgesetzt werden. Hierdurch kann die Formdichtung den Innenraum vollständig umschließen und diesen zur Kassette fluidsicher abdichten. Durch die symmetrische Ausbildung der Formdichtungskomponenten ergeben sich im Aufbau keine Probleme. Die Anordnung der Gastauscher-Verschlüsse in entsprechenden Aussparungen verringert die Komplexität der Formdichtung und der Kassette. Weiterhin wird eine Anordnung der Gastauscher-Elemente zumindest teilweise vorgegeben, so dass das Einsetzen dieser Elemente unterstützt wird.

Ebenfalls wird durch die Aufnahme in den Aussparungen bzw. Öffnungen eine verbesserte Kühlung ermöglicht, was aufgrund eines unterschiedlichen Materials erforderlich sein kann, um eine homogene Temperaturverteilung und Abkühlung ohne Blasenbildung zu bewirken.

Die Kassette kann weiterhin zum Erleichtern des Aufbaus eine Zentriervorrichtung aufweisen, beispielsweise Zentrierhülsen, welche das Anordnen und Aufstecken der Kassettenbauteile vereinfacht und eine relative Bewegung beschränkt oder verhindert. Weiterhin können die Kassettenbauteile optional mittels eines Scharniers aneinandergekoppelt sein und/oder es können Fixierschrauben vorgesehen sein, um die Kassettenbauteile miteinander zu befestigen und die Formdichtung hinreichend einzuklemmen, so dass diese fluidsicher abgedichtet ist.

Das untere Kassettenbauteil kann weiterhin zur stabilen Montage auf einer Zentrifuge konfiguriert sein, während das obere Kassettenbauteil den Vergussraum schließt und bevorzugt thermisch mit der Öffnung koppelt.

Die Aussparungen ermöglichen eine vereinfachte Abtrennung der Kassette von der Formdichtung sowie eine vereinfachte Abtrennung des vergossenen Gastauschers von der Formdichtung, zumal der Gastauscher über den Verschluss aus der Formdichtung gedrückt werden kann.

Weiterhin wird ein Verfahren zum Herstellen eines Vergusses für einen Gastauscher unter Einwirkung einer Zentrifugalkraft offenbart, welches folgende Schritte umfasst:
- Bereitstellen einer Kassette und Einsetzen von Gastauscher-Elementen in einen inneren Hohlraum der Kassette;
- Bereitstellen eines Verteilers mit mindestens einem durchgehenden Kanal und Befestigen des Verteilers auf der Kassette, so dass eine Öffnung des Verteilers fluidisch über den Kanal mit dem Hohlraum verbunden ist, um eine Vergussvorrichtung zum Herstellen eines Vergusses für einen Gastauscher zu bilden; und
- Eingeben eines Vergussmaterials in die Öffnung unter Einwirkung einer Zentrifugalkraft,
wobei das Befestigen des Verteilers das Zusammensetzen von Verteilerkomponenten umfasst, um die Öffnung des Verteilers zu definieren und zwischen aneinandergrenzenden Abschnitten der Verteilerkomponenten den Kanal zu bilden und wobei das Verfahren unter Verwendung der erfindungsgemäßen Vergussvorrichtung ausgeführt wird.

Die Verteilerkomponenten können im Aufbau nacheinander an der Kassette befestigt und beispielsweise mittels Schrauben gesichert werden. Weiterhin kann eine Abdichtung zwischen den Verteilerkomponenten verlangen, dass ein Klemmring um die Verteilerkomponenten angelegt und leicht angezogen wird, um die Grenzflächen der Verteilerkomponenten einzuklemmen bzw. zusammenzudrücken. Zwischen dem mindestens einen Kanal und der Kassette kann weiterhin eine Flachdichtung vorgesehen sein, welche bei der Befestigung des Verteilers eine hinreichende Abdichtung zwischen der Kassette und dem Verteiler bereitstellt. Eine solche Flachdichtung kann beispielsweise als O-Ring in bzw. auf eine Nut einer Bohrung eingesetzt bzw. aufgesetzt werden.

Zum Herstellen des Vergusses wird die Vergussvorrichtung auf die Zentrifuge gespannt, die sich um Mittelachse der Vergussvorrichtung dreht. Bei laufender Zentrifuge wird flüssiges Vergussmaterial über die Öffnung eingefüllt bzw. eingegeben. Zum Einfüllen des Materials kann/können beispielsweise eine oder mehrere Düsen vorgesehen sein, welche das Vergussmaterial punktuell in die Öffnung einspritzen, beispielsweise in einen trichterförmigen Füllbereich der Öffnung. Unter Einwirkung der Zentrifugalkraft wird das Vergussmaterial anschließend über den mindestens einen Kanal verteilt, so dass das Vergussmaterial über optionale Bohrungen der Kassette in den inneren Hohlraum gelangen kann.

Das flüssige Vergussmaterial kann weiterhin temperiert werden, so dass eine Aushärtung des Materials optimal verlaufen kann. Beispielsweise können die Vergussvorrichtung oder einzelne Abschnitte der Vergussvorrichtung aktiv beheizt oder gekühlt werden, so dass eine Aushärtung oder Polymerisierung homogen verläuft. So kann als Vergussmaterial beispielsweise Polyurethan verwendet werden und durch die Temperierung eine Blasenbildung vermieden werden. Dadurch wird das Risiko von Undichtigkeiten im Gastauscher reduziert.

Nach dem Aushärten des Vergussmaterials kann es aufgrund seiner Festigkeit nicht aus den jeweiligen Kanälen des Trichters gezogen werden. Durch die Aufteilung oder Abtrennung der Verteilerkomponenten und einer optionalen Antihaftbeschichtung im Verteiler kann das Vergussmaterial sich jedoch ablösen, so dass das Material aus den jeweiligen Kanälen entfernt werden kann und die Kanäle bzw. Teilkanäle jeweils gereinigt werden können. Um die Abtrennung von der Kassette zu erleichtern, können die Verteilerkomponenten weiterhin an dem Bereich, der mit der Kassette verbunden wird, eine oder mehrere Aussparungen aufweisen, beispielsweise zwei keilförmige Aussparungen. Das Einführen eines entsprechenden keilförmigen Elements und/oder einer Gabel kann dann eine Presspassung und/oder eine Hebelkraft bewirken, welche die Abtrennung der jeweiligen Verteilerkomponenten von der Kassette erleichtert, während das ausgehärtete Vergussmaterial zurückbleibt.

Bevorzugt umfasst das Zusammensetzen der Verteilerkomponenten das Anbringen einer inneren Dichtung und einer äußeren Dichtung an gegenüberliegenden Seiten des jeweiligen Kanals an jeder Verteilerkomponente, um den jeweiligen Kanal im zusammengesetzten Zustand des Verteilers fluidisch abzudichten.

So kann jede Grenzfläche der Verteilerkomponenten mit einer Dichtung versehen sein, wobei eine Dichtschnur auf einer Seite der Verteilerkomponente bzw. an einer Grenzfläche in eine an der Grenzfläche vorgesehene Dichtnut eingelegt werden kann, wobei die gegenüberliegende Grenzfläche der jeweils anderen Verteilerkomponente glatt ist und somit eine optimale Dichtfläche bereitstellt. Die Dichtschnüre erstrecken sich bevorzugt von der Öffnung bis zur Kassette bzw. einer Bohrung der Kassette, wobei die Kassette an dieser Stelle ebenfalls eine Dichtung in Form einer Flachdichtung aufweisen kann, wie vorstehend beschrieben. Derart wird sichergestellt, dass das in die Öffnung eingeführte Vergussmaterial über den Kanal in den inneren Hohlraum der Kassette fließen kann, ohne aufgrund der Zentrifugalkräfte aus der Vergussvorrichtung auszutreten.

Weiterhin kann das Bereitstellen der Kassette einen oder mehrere, bevorzugt alle der folgenden Schritte umfassen:
- Bereitstellen einer Formdichtung, welche aus zwei koppelbaren Formdichtungskomponenten ausgebildet ist, wobei die Kassette verschließbar und zum Aufnehmen der Formdichtung ausgebildet ist und wobei die Kassette aus einem in Richtung einer Zentrifugalachse der Vergussvorrichtung oberen und unteren koppelbaren Kassettenbauteil ausgebildet ist;
- Einsetzen einer ersten Formdichtungskomponente in das untere Kassettenbauteil und Einsetzen der Gastauscher-Elemente in die erste Formdichtungskomponente;
- Aufsetzen der zweiten Verteilerkomponente auf die erste Formdichtungskomponente; und
- Aufsetzen des oberen Kassettenbauteils auf die zweite Formdichtungskomponente, so dass die Formdichtung den Innenraum vollständig umschließt und diesen bis zum mindestens einen Kanal fluidsicher abdichtet.

Zur vereinfachten Handhabung beim Aufbau kann eine Zentriervorrichtung verwendet werden, beispielsweise Zentrierhülsen, welche in das untere Kassettenbauteil eingesetzt werden und auf die das obere Kassettenbauteil aufgesteckt wird. Die Kassette kann anschließend mit Schrauben geschlossen bzw. fixiert werden.

Die Formdichtungskomponenten können im Wesentlichen identisch ausgebildet sein, wobei eine obere Formdichtungskomponente an der Angussstelle bzw. im Bereich des jeweiligen Kanals oder der optionalen Bohrung eine Dichtlippe umfasst, welche vorher durchgestochen oder ausgestochen wird, um eine Fluidverbindung zwischen dem jeweiligen Kanal und dem inneren Hohlraum zu ermöglichen. Derart wird eine abgedichtete Trennung zur Kassette bereitgestellt. Dadurch können nach der Aushärtung die Kassette von der Formdichtung und die Formdichtung von dem vergossenen Gastauscher ohne weiteres abgezogen bzw. abgetrennt werden.

Erfindungsgemäß ist die im Verfahren eingesetzte Vergussvorrichtung eine erfindungsgemäße Vergussvorrichtung, wie vorstehend beschrieben.

### Kurze Beschreibung der Figuren

Bevorzugte weitere Ausführungsformen der Erfindung werden durch die nachfolgende Beschreibung der Figuren näher erläutert. Dabei zeigen:
Figur 1 ist eine perspektivische Darstellung einer Vergussvorrichtung gemäß der Erfindung in zusammengesetztem Zustand;
Figur 2 ist eine perspektivische Darstellung der Vergussvorrichtung gemäß Figur 1 mit einer fehlenden Verteilerkomponente;
Figur 3 ist eine Schnittdarstellung der Vergussvorrichtung gemäß Figur 1;
Figur 4 ist eine perspektivische Schnittdarstellung der Vergussvorrichtung gemäß Figur 3;
Figur 5 ist eine perspektivische Darstellung einer Kassette mit einer Formdichtung und einer Aussparung gemäß der Erfindung;
Figur 6 ist eine obere perspektivische Darstellung einer Formdichtungskomponente für eine Kassette mit einer Aussparung gemäß der Erfindung;
Figur 7 ist eine untere perspektivische Darstellung der Formdichtungskomponente gemäß Figur 6;
Figur 8 ist eine obere perspektivische Darstellung der Formdichtungskomponente gemäß Figur 6 mit einem eingesetzten Gastauscher-Verschluss;
Figur 9 ist eine Seitenansicht einer Formdichtung mit eingesetzten Gastauscher-Verschlüssen gemäß der Erfindung;
Figur 10 ist eine perspektivische Darstellung eines unteren Kassettenbauteils für die Kassette gemäß der Erfindung;
Figur 11 ist eine perspektivische Darstellung des unteren Kassettenbauteils gemäß Figur 10 mit eingesetzter Formdichtungskomponente und Gastauscher-Verschluss;
Figur 12 ist eine perspektivische Darstellung eines oberen Kassettenbauteils für die Kassette gemäß der Erfindung;
Figur 13 ist eine perspektivische Schnittdarstellung des oberen Kassettenbauteils gemäß Figur 12;
Figur 14 ist eine perspektivische Schnittdarstellung der Kassette gemäß Figur 5 mit einer verbleibenden Verteilerkomponente;
Figur 15 ist eine Detailansicht einer Dichtung an einer Grenzfläche einer Verteilerkomponente gemäß der Erfindung;
Figur 16 ist eine weitere Detailansicht der Dichtung gemäß Figur 15; und
Figur 17 ist eine Detailansicht einer Abdichtung des Kanals an einer Kassettenoberfläche gemäß der Erfindung.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird weitgehend verzichtet, um Redundanzen zu vermeiden.

Anhand der Figuren werden die einzelnen Komponenten der Vergussvorrichtung beschrieben, wobei die jeweiligen Komponenten zur besseren Übersicht nicht in allen Figuren gezeigt sind und wobei optionale Komponenten in den Ausführungsformen vorhanden sein können.

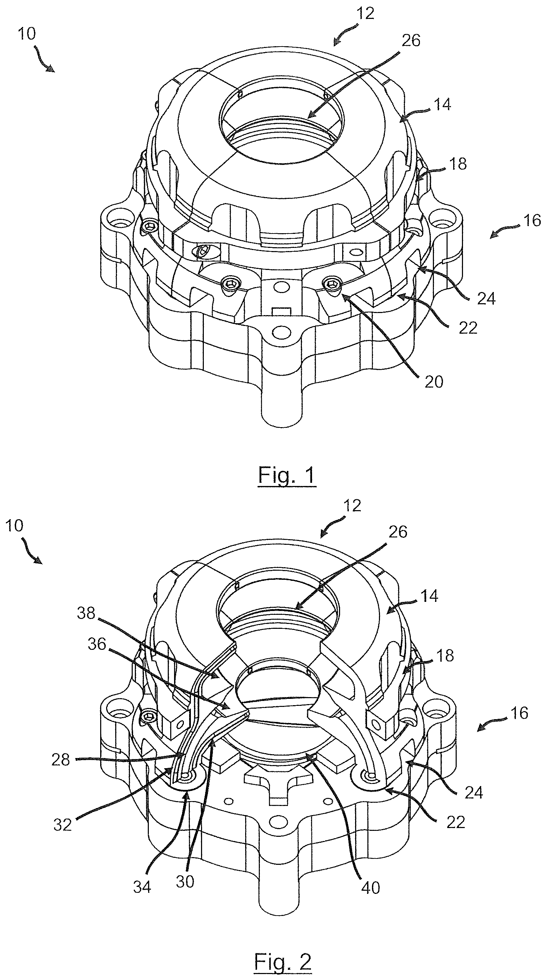

In Figur 1 wird eine Vergussvorrichtung 10 gemäß der Erfindung in zusammengesetztem Zustand in einer perspektivischen Darstellung gezeigt. Die Vergussvorrichtung 10 umfasst dabei einen Verteiler 12, der dazu eingerichtet ist, ein Vergussmaterial über eine Öffnung 26 zu empfangen und über einen oder mehrere Kanäle im Inneren der Vergussvorrichtung 10 bis zu einer Kassette zu verteilen, so dass das Vergussmaterial einen Verguss für in der Kassette 16 enthaltene Gastauscher-Elemente (nicht gezeigt) bilden kann.

Der Verteiler 12 ist aus mehreren Verteilerkomponenten 14 gebildet. Gemäß dieser Ausführungsform sind vier Verteilerkomponenten 14 vorgesehen, welche zusammen einen zylinderförmigen Verteiler 12 bilden und dabei die Öffnung 26 definieren. Es kann jedoch auch eine alternative Anzahl von Verteilerkomponenten 14 vorgesehen sein, beispielsweise zwei oder drei. Die äußere Form des Verteilers 12 ist nicht auf eine Zylinderform beschränkt, sondern kann beispielsweise auch ellipsoid oder im Wesentlichen rechteckig ausgebildet sein.

Die Verteilerkomponenten 14 sind mittels eines Klemmrings 18 miteinander verbunden, so dass diese an entsprechenden Grenzflächen 22 abgedichtet sind. Wie nachstehend erläutert, werden in dieser Ausführungsform Kanäle durch die Grenzflächen 22 ausgebildet, welche das über die Öffnung 26 empfangene Vergussmaterial zur Kassette 16 führen. In dieser Ausführungsform sind entsprechend vier Kanäle vorgesehen, welche umlaufend angeordnet und gleichmäßig voneinander beabstandet sind, so dass die in der Kassette 16 enthaltenen Gastauscher-Elemente von vier Seiten mit dem Vergussmaterial umgeben wird.

Zur Befestigung des Verteilers 12 bzw. der Verteilerkomponenten 14 an der Kassette 16 sind weiterhin Fixierschrauben 20 vorgesehen, welche eine lösbare Verbindung bereitstellen. Entsprechend können die Verteilerkomponenten 14 nachfolgend auf die Kassette 16 aufgeschraubt und nach einem Herstellungsvorgang auch wieder abgeschraubt und von der Kassette 16 abgetrennt werden.

Um das Abtrennen der Verteilerkomponenten 14 zu erleichtern, sind Aussparungen 24 vorgesehen, welche mit einer Trennvorrichtung, beispielsweise einer keilförmigen Trennvorrichtung, in Eingriff gebracht werden können, um die Verteilerkomponenten 14 von der Kassette 16 zu entfernen.

In Figur 2 ist die Vergussvorrichtung 10 mit einer fehlenden (nicht gezeigten) Verteilerkomponente 14 gezeigt, wobei die Grenzflächen 22 und das Innere des Verteilers 12 freigelegt sind. Entsprechend ist ein Teil des Kanals 28 gezeigt, welcher in zusammengesetzten Zustand des Verteilers mit einem entsprechenden Teil des Kanals 28 einer angrenzenden Grenzfläche 22 der jeweils anderen Verteilerkomponente 14 den Kanal 28 bildet. Mit anderen Worten kann jede Grenzfläche 22 einen halben Kanal definieren, welche so angeordnet ist, dass beim Zusammensetzen des Verteilers ein Kanal 28 durch benachbarte Grenzflächen 22 von zwei Verteilerkomponenten 14 ausgebildet wird.

Der Kanal 28 ist weiterhin von einer Dichtung umgeben, um zu verhindern, dass Vergussmaterial während der Herstellung aufgrund der einwirkenden Zentrifugalkräften nach außen austreten kann. Die Dichtung wird von einer inneren Dichtung 30 und einer äußeren Dichtung 32 bereitgestellt, welche an gegenüberliegenden Seiten des Kanal 28 angeordnet sind. Die Dichtungen 30, 32 erstrecken sich von der Öffnung 26 bis zum Ende des Kanals 28 bzw. bis hin zu einer Bohrung der Kassette (nicht gezeigt). An diesem Ende ist weiterhin eine Flachdichtung 34 vorgesehen, welche in der Bohrung eingelegt ist und den Kanal ringsum umgibt. Somit ist der Kanal 28 fluidsicher abgedichtet.

Das Vergussmaterial kann ohne Verluste oder Beeinträchtigung der Herstellung bis zu den Gastauscher-Elementen gelangen. Um das Einfüllen zu vereinfachen, ist die Öffnung 26 stromaufwärts der jeweiligen Kanälen 28 trichterförmig ausgebildet, so dass ein Füllbereich 36 definiert wird. Durch die Trichterform, welche ringsum durchgehend ausgebildet sein kann, kann das Vergussmaterial auch beim Abprallen sicher in den Kanal 28 geführt werden.

Weiterhin ist oberhalb des Füllbereichs 36 ein Überlauf 38 vorgesehen, welcher an die Geometrie des Füllbereichs 36 sowie den Innenraum der Kassette 16 angepasst ist. Der Überlauf 38 kann aufgrund der Trichterform des Füllbereichs 36 und dessen Anordnung eine Führung von überschüssigem Vergussmaterial bewirken, so dass das überschüssige Vergussmaterial in den Überlauf 38 läuft und dort gesammelt wird, wenn das Vergussmaterial in dem inneren Hohlraum der Kassette 16 so weit eingefüllt ist, dass es in radialer Richtung den zur Öffnung 26 ausgerichteten Rand des Füllbereichs 36 erreicht hat. Mit anderen Worten sind der Füllbereich 36 und der Überlauf 38 so geformt, dass diese eine größere radiale Beabstandung zur Mitte der Kassette 16 als inneren Toleranzbereich des herzustellenden Vergusses umfassen.

Die Öffnung 26 erstreckt sich bis zur äußeren Oberfläche der Kassette 16 bzw. bis zur Oberseite der Kassette 16 und ermöglicht durch den Austausch mit der Umgebung beispielsweise eine Kühlung von deren Oberseite. Hierdurch kann die Kassette 16 gleichmäßig temperiert werden, so dass eine Blasenbildung aufgrund einer unregelmäßigen oder auch ungleichmäßigen Abkühlung verhindert wird. Auch kann die Öffnung eine direkte Kühlung der Gastauscher-Elemente bewirken, wie durch den beispielhaften Verschluss 40, der in einer Aussparung der Kassette 16 aufgenommen ist, gezeigt.

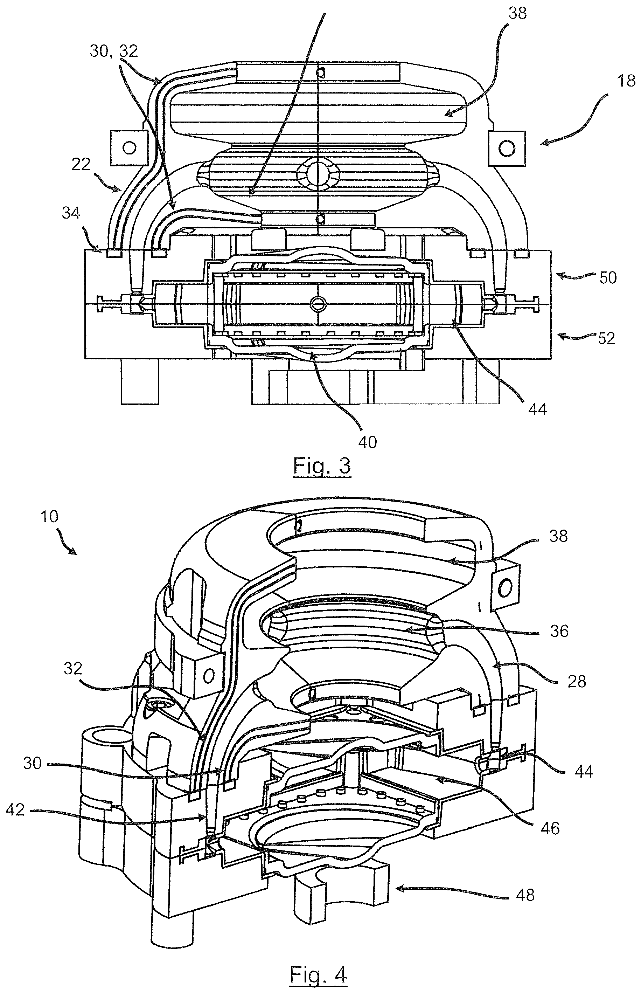

In der Schnittdarstellung der Vergussvorrichtung 10 in Figur 3 ist das Innere der Kassette 16 gezeigt. Es umfasst die Kassette 16 zwei Kassettenbauteile 50, 52, nämlich ein oberes Kassettenbauteil 50 und ein unteres Kassettenbauteil 52, welche miteinander verbunden und verschließbar ausgebildet sind. Die Kassettenbauteile 50, 52 können somit voneinander getrennt bzw. geöffnet werden, um eine Formdichtung 44 aufzunehmen, welche den Innenraum der Kassette 16 verkleidet und entsprechend eingeklemmt wird, um eine fluidische Abdichtung eines inneren Hohlraums zur Kassette 16 zu ermöglichen.

Dieser Aufbau wird weiter in Figur 4 gezeigt. Der innere Hohlraum 46 der Formdichtung 44 ist dargestellt. Der innere Hohlraum 46 ist fluidisch mit den Kanälen 28 über jeweilige Bohrungen 42 der Kassette verbunden. Es wird jedoch verhindert, dass Vergussmaterial von den Kanälen 28 in die Kassette bzw. in das Innere der Kassette eindringen kann, beispielsweise durch Dichtlippen der Formdichtung 44, die im Bereich der Bohrungen 42 angeordnet sind. Die Dichtlippen (nicht gezeigt) werden durch den Zusammenbau der Kassette eingeklemmt und bilden eine Dichtung zur Bohrung 42. So gelangt Vergussmaterial von dem jeweiligen Kanal 28 über die Bohrung 42 nur in den inneren Hohlraum 46 der Formdichtung 44. Die Gastauscher-Elemente, vorliegend zwei gegenüberliegend angeordnete Verschlüsse des Gastauschers, sind ebenfalls in der Formdichtung 44 angeordnet und werden derart bis zum inneren Durchmesser des Füllbereichs 36 vom Vergussmaterial umgeben. Hierdurch wird ein Kontakt des Vergussmaterials mit der Kassette verhindert, was die Reinigung und Trennung der Kassettenbauteile erheblich erleichtert. Die Kassette umfasst weiterhin am unteren Bereich einen Zentrifugenaufsatz 48, welcher das Montieren in einer Zentrifuge erleichtert und eine ortsfeste Fixierung ermöglicht.

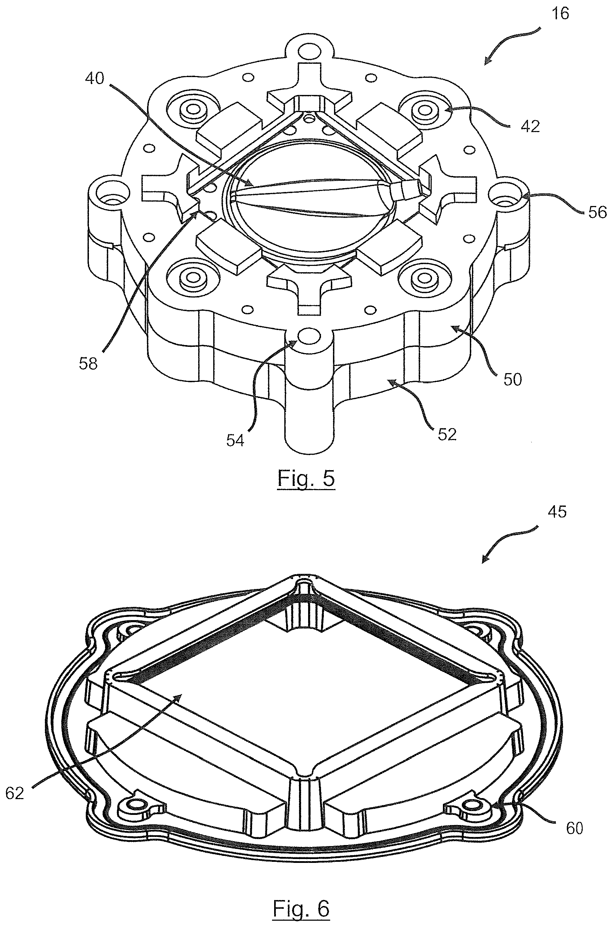

Die Kassette 16 ist im Detail in der perspektivischen Darstellung gemäß Figur 5 gezeigt, wobei die Kassette 16 mit einer Formdichtung und einer Öffnung 58 im oberen Kassettenbauteil 50 gezeigt ist. Die Öffnung 58 ermöglicht, dass ein Gastauscher-Element bzw. ein Verschluss 40 aus der Kassette hinausragen und durch die Öffnung des Verteilers belüftet werden können.

Zusätzlich zu der Öffnung 58 umfasst das obere Kassettenbauteil 50 neben den Bohrungen 42 eine Zentriervorrichtung 54, in die beispielsweise Zentrierhülsen eingesetzt werden können, so dass das obere Kassettenbauteil 50 einfach auf das untere Kassettenbauteil 52 aufgesteckt und entsprechend ausgerichtet werden kann. Weiterhin sind zwei Fixiervorrichtungen 56 vorgesehen, mittels welcher die Kassettenbauteile 50, 52 miteinander verbunden werden können, beispielsweise über entsprechende Schrauben.

Die Formdichtung 44 wird bevorzugt aus zwei symmetrischen Formdichtungskomponenten 45 ausgebildet, wie in den Figuren 6 bis 9 gezeigt. Entsprechend weist die Formdichtungskomponente 45 analog zur Kassette eine Öffnung 62 auf, in der beispielsweise ein Verschluss des Gastauschers aufgenommen werden kann. Dadurch kann die Komplexität der Formdichtungskomponente 45 reduziert werden. Weiterhin wird hierdurch eine vereinfachte Abtrennung der Formdichtungskomponente 45 von den vergossenen Gastauscher-Elementen nach der Aushärtung des Vergussmaterials ermöglicht. Beispielsweise kann ein Gastauscher über den hinausragenden Verschluss aus der Formdichtungskomponente 45 gedrückt werden.

Die Formdichtungskomponente 45 umfasst weiterhin eine der Anzahl der Kanäle entsprechende Anzahl an Dichtlippen bzw. Angussstellen 60. Die Angussstellen 60 sind derart angeordnet, dass diese mit einer Bohrung der Kassette sowie dem Ausgang des jeweiligen Kanals überlappen, so dass eine fluidische Abdichtung zur Kassette bereitgestellt wird. Dadurch, dass die Formdichtungskomponenten 45 symmetrisch ausgebildet sind und eine fluidische Verbindung für die entsprechende untere Formdichtungskomponente 45 nicht vorgesehen ist, können die Angussstellen 60 zunächst verschlossen und vor dem Einlegen bzw. Einsetzen der oberen Formdichtungskomponente 45 ausgestochen, durchstochen oder perforiert werden, um die Fluidverbindung zwischen den Kanälen und dem inneren Hohlraum bereitzustellen.

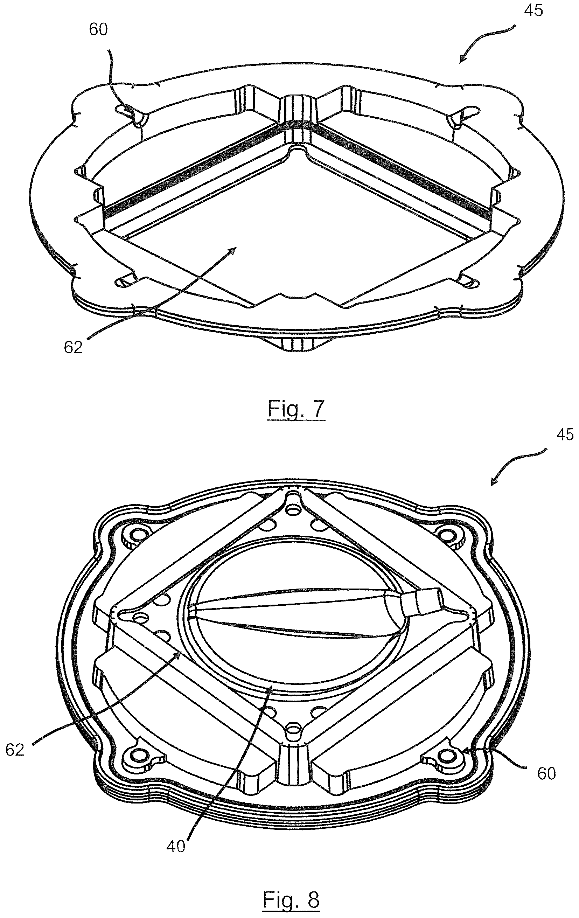

Entsprechend ist in Figur 7 eine untere Formdichtungskomponente 45 gezeigt, wobei die Angussstellen 60 nicht ausgestochen sind, ein kurzer Zulauf zum inneren Hohlraum jedoch ebenfalls vorhanden ist.

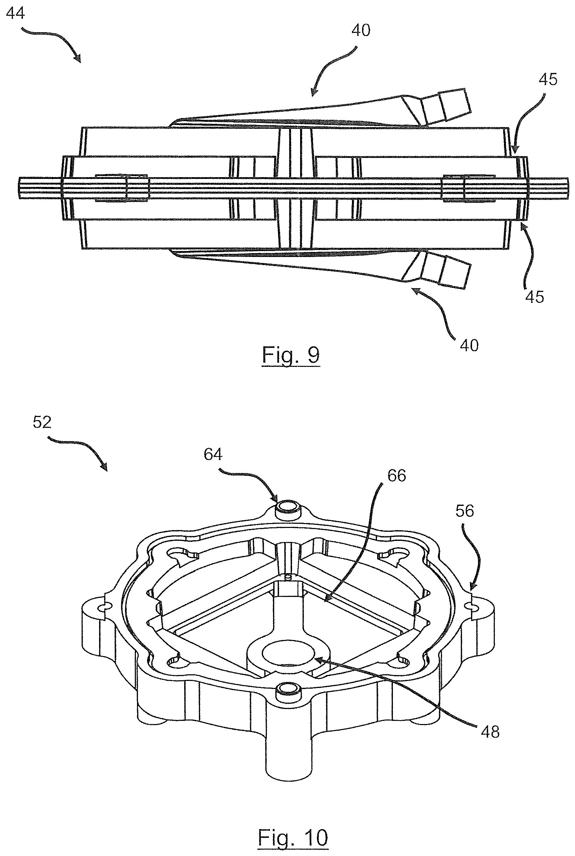

Weiterhin zeigt Figur 8 die obere Formdichtungskomponente 45 mit einem Verschluss 40, der in der Öffnung 62 eingesetzt ist, wobei die Angussstellen 60 noch nicht ausgestochen sind. Die untere Formdichtungskomponente 45 kann mit einem Verschluss 40 versehen sein, wobei die Formdichtungskomponenten 45, wenn diese zusammengesetzt werden, gemeinsam die Formdichtung 44 bilden, wie in Figur 9 in einer Seitenansicht gezeigt.

Die untere Formdichtungskomponente 45 kann von einem unteren Kassettenbauteil 52 durch eine entsprechende Ausgestaltung aufgenommen werden, wie in Figur 10 in einer perspektivischen Darstellung gezeigt. Das untere Kassettenbauteil 52 umfasst in dieser Ausführungsform neben der Fixiervorrichtung 56 einen Zentrifugenaufsatz 48, welcher das Montieren der Vergussvorrichtung in einer Zentrifuge erleichtert und eine ortsfeste Fixierung ermöglicht. Auch sind in die Zentriervorrichtung entsprechende Zentrierhülsen 64 eingesetzt, wodurch das obere Kassettenbauteil auf das untere Kassettenbauteil 52 aufgesteckt und gleichzeitig bedarfsgerecht ausgerichtet werden kann.

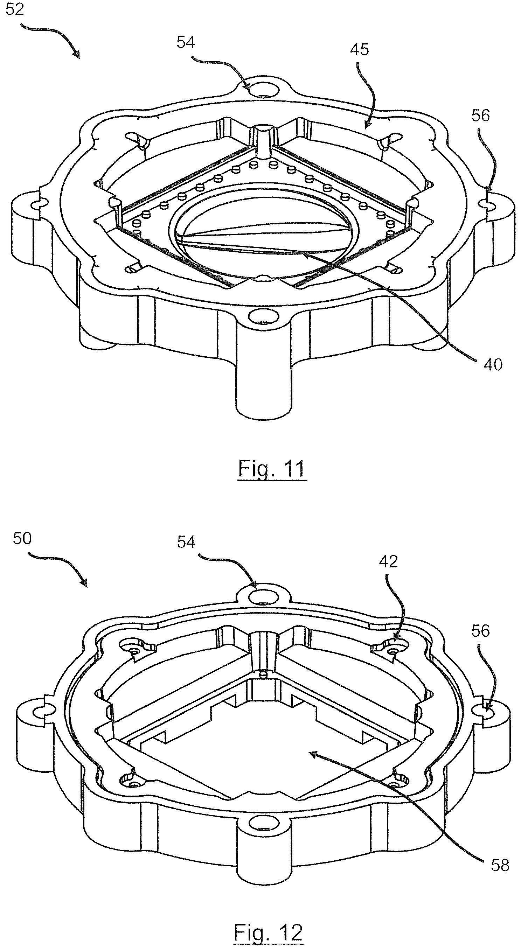

Das untere Kassettenbauteil 52 umfasst, wie das obere Kassettenbauteil, eine Öffnung 66 zur Aufnahme eines Verschlusses. Hierdurch kann auch an der Unterseite der Kassette eine Belüftung des zu vergießenden Gastauschers erfolgen, so dass eine verbesserte Temperaturverteilung erreicht wird. Dies ist in Figur 11 gezeigt. Eine Formdichtungskomponente 45 ist in dem unteren Kassettenbauteil 52 eingesetzt und ein Verschluss 40 ist von der Öffnung der

Formdichtungskomponente 45 aufgenommen. In den Zentriervorrichtungen 54 sind in dieser Figur noch keine Zentrierhülsen eingesetzt. Aus dieser Figur wird ersichtlich, dass eine Fluidverbindung an der unteren Seite nicht möglich ist, da keine Angussstellen oder Bohrungen vorhanden sind.

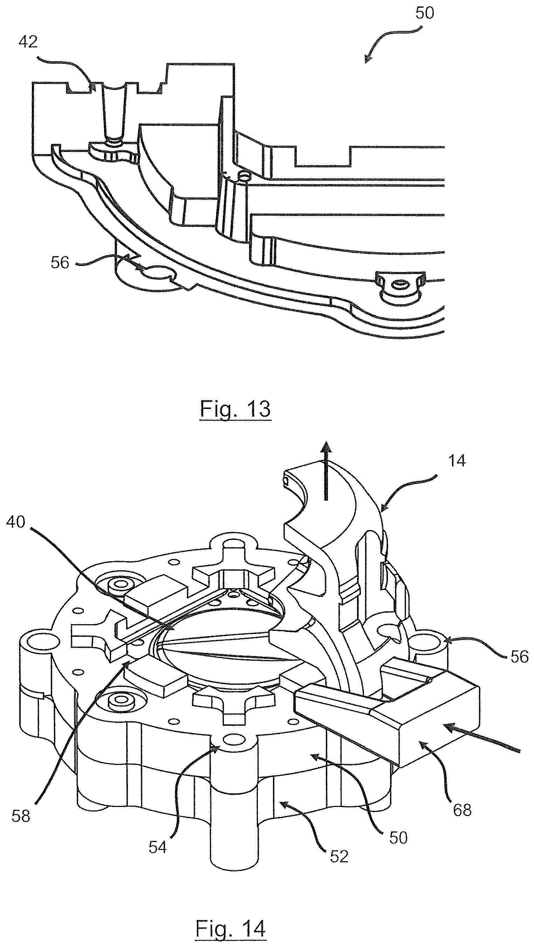

Dies steht im Gegensatz zu den im oberen Kassettenbauteil 50 vorhandenen Bohrungen 42, wie in Figur 12 gezeigt. Die Bohrungen 42 sind im größeren Detail in Figur 13 gezeigt. Entsprechend können die Bohrungen 42 im oberen Kassettenbauteil 50 einen Zulauf für das eingespeiste Vergussmaterial von dem jeweiligen Kanal bis zum inneren Hohlraum der Formdichtung bereitstellen. Die Bohrung 42 stellt nur einen verhältnismäßig kleinen Zulauf bereit, welcher nach der Herstellung einfach gereinigt werden kann. Zur Abdichtung kann die Bohrung 42 an der oberen Seite mit einer Flachdichtung versehen sein, so dass der jeweilige Kanal beispielsweise ringsum abgedichtet wird.

Die Abtrennung zwischen der Kassette und dem jeweiligen Kanal wird bevorzugt durch eine entsprechende Aussparung am unteren Bereich der jeweiligen Verteilerkomponenten 14 erleichtert. Beispielsweise kann die Aussparung keilförmig ausgebildet sein, so dass eine keilförmige Trennvorrichtung 68 in die Aussparung eingeführt werden kann und eine Trennbewegung bewirkt, wie in Figur 14 mit Pfeilen angedeutet. So kann die Trennvorrichtung 68 beispielsweise als Gabel ausgebildet sein, wobei die Gabel in zwei nebeneinander angeordneten Aussparungen von zwei Verteilerkomponenten eingreift. Derart kann der Verteiler bzw. können die Verteilerkomponenten 14 nachfolgend von der Kassette bzw. dem oberen Kassettenbauteil 50 abgelöst und anschließend gereinigt werden.

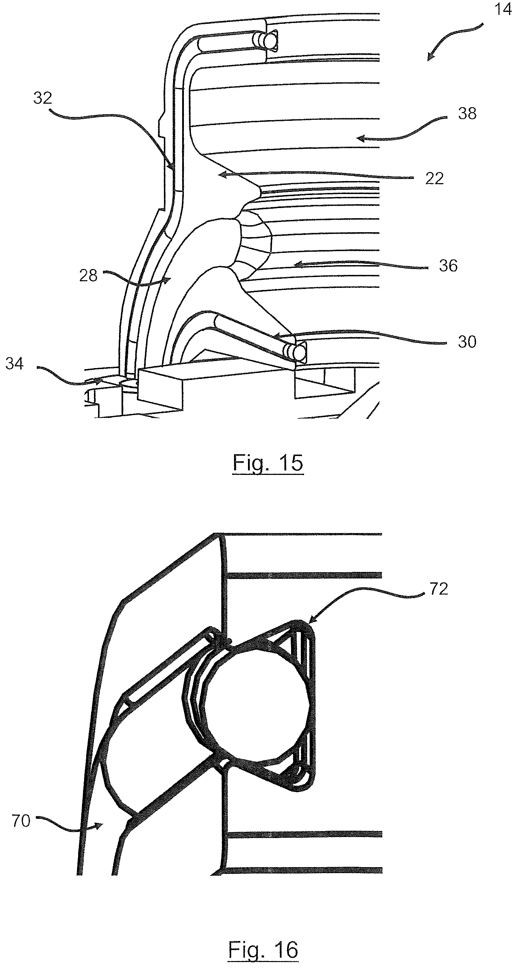

In den Figuren 15 bis 17 sind vorteilhafte Dichtungen der Vergussvorrichtung in Detailansichten gezeigt. Entsprechend zeigt Figur 15 eine Detailansicht einer Grenzfläche 22 einer Verteilerkomponente 14, wobei der Kanal 28 von einer inneren Dichtung 30 und einer äußeren Dichtung 32 umgeben wird und somit fluidisch gegenüber der Umgebung abgedichtet werden kann. Die äußere Dichtung 32 verläuft von einem oberen Bereich der Öffnung und dem Überlauf 38 bis zum unteren Ende bzw. stromabwärts gelegenen Ende des Kanals 28, so dass die äußere Dichtschnur mögliche Vergussmaterial enthaltende Komponenten des Verteilers umgibt. Weiterhin dichtet die innere Dichtung 30 den Kanal 28 zur Kassette und zum unteren Abschnitt des Füllbereichs 36 ab. Dort wo der jeweilige Kanal 28 in eine Bohrung der Kassette mündet, wird der Kanal 28 bevorzugt von einer Flachdichtung 34 umgeben. Das Vergussmaterial kann dann vom Füllbereich 36 sicher und ohne Austreten in die Bohrung bzw. den inneren Hohlraum der Formdichtung gelangen.

Wie in Figur 16 gezeigt, sind die innere und äußere Dichtung 30, 32 weiterhin an den Grenzflächen 22 mittels einer Dichtnut 72 befestigt, wobei eine entsprechende Dichtschnur 70 in der Dichtnut 72 eingelegt ist. Die Dichtschnur 70 besteht aus einem elastischen Material, so dass die Dichtschnur 70 mittels einer Presspassung in die Dichtnut 72 eingesetzt werden kann. Die angrenzende Grenzfläche 22 der angekoppelten Verteilerkomponente kann ohne Dichtnut 72 ausgebildet sein und eine glatte Fläche aufweisen, so dass die Dichtschnur 70 beim Zusammensetzen des Verteilers bzw. durch Einspannen der Verteilerkomponenten an die Fläche gedrückt wird und eine fluidsichere Abdichtung erzielt wird.

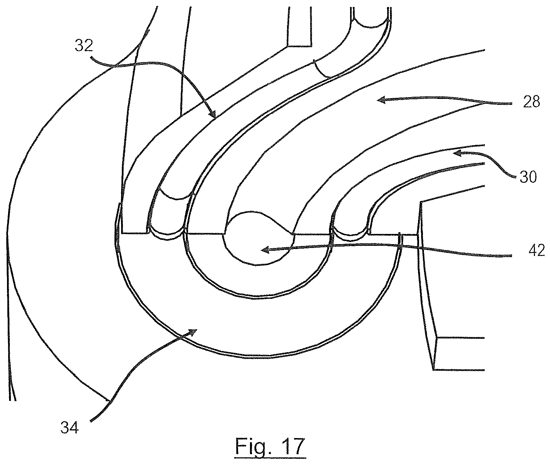

Eine fluidsichere Abdichtung zur Kassette kann auch durch eine Flachdichtung 34 bereitgestellt werden, welche am oberen Rand einer Bohrung 42 eingelegt ist und den jeweiligen Kanal 28 umgibt, wie in Figur 17 gezeigt. Die Flachdichtung 34 kann mit der inneren Dichtung 30 und der äußeren Dichtung überlappen, was nicht nur eine verbesserte Abdichtung, sondern auch eine einfachere Abtrennung der Verteilerkomponenten ermöglicht. Weiterhin können die Verteilerkomponenten dadurch kleiner dimensioniert werden, um diese kompakt auszugestalten und die Herstellungskosten zu reduzieren.

### Bezugszeichenliste

- 10: Vergussvorrichtung
- 12: Verteiler
- 14: Verteilerkomponente
- 16: Kassette
- 18: Klemmring
- 20: Fixierschraube
- 22: Grenzfläche
- 24: Aussparung
- 26: Öffnung
- 28: Kanal
- 30: innere Dichtung
- 32: äußere Dichtung
- 34: Flachdichtung
- 36: trichterförmiger Füllbereich
- 38: Überlauf
- 40: Gasaustauscher-Element bzw. Verschluss
- 42: Bohrung
- 44: Formdichtung
- 45: Formdichtungskomponente
- 46: innerer Hohlraum
- 48: Zentrifugenaufsatz
- 50: oberes Kassettenbauteil
- 52: unteres Kassettenbauteil
- 54: Zentriervorrichtung
- 56: Fixiervorrichtung
- 58: Öffnung
- 60: Dichtlippe oder Angussstelle
- 62: Öffnung
- 64: Zentrierhülse
- 66: Öffnung
- 68: keilförmige Trennvorrichtung
- 70: Dichtschnur
- 72: Dichtnut

## Patentansprüche

1. Vergussvorrichtung (10) zum Herstellen eines Vergusses für einen Gastauscher unter Einwirkung einer Zentrifugalkraft, umfassend:
- einen Verteiler (12), welcher eine Öffnung (26) und mindestens einen durchgehenden Kanal (28) umfasst und dazu ausgelegt ist, ein fluidisches Vergussmaterial über die Öffnung (26) zu empfangen und über den mindestens einen Kanal (28) zu führen, wobei der Verteiler (12) mindestens zwei Verteilerkomponenten (14) umfasst, welche im zusammengesetzten Zustand des Verteilers (12) die Öffnung (26) definieren, auslaufsicher miteinander verbunden sind und zwischen aneinandergrenzenden Bereichen den mindestens einen Kanal (28) bilden; und
- eine Kassette (16), welche einen inneren Hohlraum (46) zum Aufnehmen von Gastauscher-Elementen (40) definiert, welcher fluidisch mit dem mindestens einen Kanal (28) verbunden ist,
**dadurch gekennzeichnet, dass**
die Vergussvorrichtung (10) eine Formdichtung (44) umfasst, wobei die Kassette (16) verschließbar und zum Aufnehmen der Formdichtung (44) ausgebildet ist, wobei die Formdichtung (44) den inneren Hohlraum (46) vollständig umschließt und diesen bis zum mindestens einen Kanal (28) fluidsicher abdichtet.

2. Vergussvorrichtung (10) nach Anspruch 1, wobei die Verteilerkomponenten (14) im zusammengesetzten Zustand des Verteilers (12) mindestens zwei durchgehende Kanäle (28) bilden.

3. Vergussvorrichtung (10) nach Anspruch 1 oder 2, wobei der Verteiler (12) aus gleichförmigen Verteilerkomponenten (14) gebildet ist.

4. Vergussvorrichtung (10) nach Anspruch 3, wobei jede Verteilerkomponente (14) im zusammengesetzten Zustand des Verteilers (12) an zwei Verteilerkomponenten (14) angrenzt und mit der angrenzenden Verteilerkomponente (14) einen Kanal (28) bildet,
wobei der Verteiler (12) bevorzugt aus drei oder vier gleichförmigen Verteilerkomponenten (14) gebildet ist.

5. Vergussvorrichtung (10) nach einem der vorhergehenden Ansprüche, wobei der Verteiler (12) aus drei oder vier gleichförmigen Verteilerkomponenten (14) gebildet ist und wobei jede Verteilerkomponente (14) im zusammengesetzten Zustand des Verteilers (12) an zwei Verteilerkomponenten (14) angrenzt und mit der angrenzenden Verteilerkomponente (14) einen Kanal (28) bildet.

6. Vergussvorrichtung (10) nach einem der vorstehenden Ansprüche, wobei die Verteilerkomponenten (14) ausgelegt sind, lösbar miteinander verbunden zu werden, und für ein reversibles Zusammensetzen des Verteilers (12) ausgelegt sind.

7. Vergussvorrichtung (10) nach einem der vorstehenden Ansprüche, wobei jede Verteilerkomponente (14) eine innere Dichtung (30) und eine äußere Dichtung (32) an gegenüberliegenden Seiten des jeweiligen Kanals (28) umfasst, welche den jeweiligen Kanal (28) im zusammengesetzten Zustand des Verteilers (12) fluidisch abdichten.

8. Vergussvorrichtung (10) nach einem der vorstehenden Ansprüche, wobei die Öffnung (26) einen trichterförmigen Füllbereich (36) für das Vergussmaterial definiert,
und/oder
wobei die Öffnung (26) bis zu einer äußeren Oberfläche der Kassette (16) verläuft und diese fluidisch mit der Umgebung verbindet und wobei die Öffnung (26) konzentrisch mit einer Zentrifugalachse der Vergussvorrichtung (10) ist.

9. Vergussvorrichtung (10) nach einem der vorstehenden Ansprüche, wobei die Verteilerkomponente (14) im zusammengesetzten Zustand des Verteilers (12) einen Überlauf (38) definiert, welcher derart angeordnet und geformt ist, dass ein Materialfluss über eine vorgegebene Grenzfläche im inneren Hohlraum (46) einen Materialfluss in den Überlauf (38) bewirkt.

10. Vergussvorrichtung (10) nach einem der vorstehenden Ansprüche, wobei zumindest der Verteiler (12) wiederverwendbar ist,
und/oder
wobei die Öffnung (26) und der mindestens eine Kanal (28) mit einer Antihaftbeschichtung versehen sind,
und/oder
wobei die Verteilerkomponenten (14) aus einem Material, umfassend Aluminium, ausgebildet sind.

11. Vergussvorrichtung (10) nach einem der vorstehenden Ansprüche,
wobei die Formdichtung (44) aus zwei symmetrisch ausgebildeten und koppelbaren Formdichtungskomponenten (45) ausgebildet ist,
und/oder
wobei die Kassette (16) aus einem in Richtung einer Zentrifugalachse der Vergussvorrichtung oberen (50) und unteren (52) koppelbaren Kassettenbauteil (50, 52) ausgebildet ist, wobei die Kassettenbauteile (50, 52) zum Aufnehmen einer jeweiligen Formdichtungskomponente (45) ausgebildet sind und wobei die Kassettenbauteile (50, 52) und die Formdichtungskomponenten (45) vorzugsweise jeweils eine Aussparung oder Öffnung (58, 62) zur Aufnahme eines Gastauscher-Verschlusses (40) umfassen, welche konzentrisch mit der Zentrifugalachse der Vergussvorrichtung (10) ist.

12. Verfahren zum Herstellen eines Vergusses für einen Gastauscher unter Einwirkung einer Zentrifugalkraft, umfassend:
- Bereitstellen einer Kassette (16) und Einsetzen von Gastauscher-Elementen (40) in einen inneren Hohlraum (46) der Kassette (16);
- Bereitstellen eines Verteilers (12) mit mindestens einem durchgehenden Kanal (28) und Befestigen des Verteilers (12) auf der Kassette (16), so dass eine Öffnung (26) des Verteilers (12) fluidisch über den Kanal (28) mit dem Hohlraum verbunden ist, um eine Vergussvorrichtung (10) zum Herstellen eines Vergusses für einen Gastauscher zu bilden; und
- Eingeben eines Vergussmaterials in die Öffnung (26) unter Einwirkung einer Zentrifugalkraft,
wobei das Befestigen des Verteilers (12) das Zusammensetzen von Verteilerkomponenten (14) umfasst, um die Öffnung (26) des Verteilers (12) zu definieren und zwischen aneinandergrenzenden Bereichen der Verteilerkomponenten (14) den Kanal (28) zu bilden,
**dadurch gekennzeichnet, dass**
das Verfahren unter Verwendung einer Vergussvorrichtung (10) nach einem der Ansprüche 1 bis 11 ausgeführt wird.

13. Verfahren nach Anspruch 12, wobei das Zusammensetzen der Verteilerkomponenten (14) das Anbringen einer inneren Dichtung (30) und einer äußeren Dichtung (32) an gegenüberliegenden Seiten des jeweiligen Kanals (28) an jeder Verteilerkomponente (14) umfasst, um den jeweiligen Kanal (28) im zusammengesetzten Zustand des Verteilers (12) fluidisch abzudichten.

14. Verfahren nach Anspruch 12 oder 13, wobei das Bereitstellen der Kassette (16) umfasst:
- Bereitstellen einer Formdichtung (44), welche aus zwei koppelbaren Formdichtungskomponenten (45) ausgebildet ist, wobei die Kassette (16) verschließbar und zum Aufnehmen der Formdichtung (44) ausgebildet ist und wobei die Kassette (16) aus einem in Richtung einer Zentrifugalachse der Vergussvorrichtung (10) oberen und unteren koppelbaren Kassettenbauteil (50, 52) ausgebildet ist;
- Einsetzen einer ersten Formdichtungskomponente (45) in den unteren Kassettenbauteil (52) und Aufsetzen der Gastauscher-Elemente (40) auf die erste Formdichtungskomponente (45);
- Aufsetzen der zweiten Formdichtungskomponente (45) auf die erste Formdichtungskomponente (45); und
- Aufsetzen des oberen Kassettenbauteils (50) auf die zweite Formdichtungskomponente (45), so dass die Formdichtung (44) den Innenraum vollständig umschließt und diesen bis zum mindestens einen Kanal (28) fluidsicher abdichtet.

## Claims

1. Casting device (10) for producing a potting for a gas exchanger under the influence of a centrifugal force, comprising:
- a distributor (12) comprising an opening (26) and at least one continuous channel (28) and which is adapted to receive a fluidic potting material via the opening (26) and to guide the potting material via the at least one channel (28), wherein the distributor (12) comprises at least two distributor components (14) which, in the assembled state of the distributor (12), define the opening (26), are connected to one another in a leak-proof manner, and form the at least one channel (28) between adjacent regions; and
- a cassette (16) defining an inner cavity (46) for receiving gas exchanger elements (40) and which inner cavity is fluidically connected to said at least one channel (28),
**characterized in that**
the casting device (10) comprises a molded seal (44), wherein the cassette (16) is closable and formed to receive the molded seal (44), wherein the molded seal (44) completely encloses the inner cavity (46) and seals the inner cavity (46) in a fluid-tight manner up to the at least one channel (28).

2. Casting device (10) according to claim 1, wherein the distributor components (14) form at least two continuous channels (28) in the assembled state of the distributor (12).

3. Casting device (10) according to claim 1 or 2, wherein the distributor (12) is formed of uniformly shaped distributor components (14).

4. Casting device (10) according to claim 3, wherein, in the assembled state of the distributor (12), each distributor component (14) is adjacent to two distributor components (14) and forms a channel (28) with the adjacent distributor component (14),
wherein the distributor (12) is preferably formed of three or four uniformly shaped distributor components (14).

5. Casting device (10) according to any one of the preceding claims, wherein the distributor (12) is formed by three or four uniformly shaped distributor components (14) and wherein each distributor (12), in an assembled state of the distributor (12), is adjacent to two distributor components (14) and forms a channel (28) with the adjacent distributor component (14).

6. Casting device (10) according to any one of the preceding claims, wherein the distributor components (14) are configured to be connectable with each other in a detachable manner and are configured for reversibly assembling the distributor (12).

7. Casting device (10) according to any one of the preceding claims, wherein each distributor component (14) comprises an inner seal (30) and an outer seal (32) on opposite sides of the respective channel (28) which fluidly or fluid-tightly seal the respective channel (28) in the assembled state of the distributor (12).

8. Casting device (10) according to any one of the preceding claims, wherein the opening (26) defines a funnel-shaped filling region (36) for the potting material,
and/or
wherein the opening (26) extends to an outer surface of the cassette (16) and fluidly connects the cassette (16) to the environment, and wherein the opening (26) is concentric with a centrifugal axis of the casting device (10).

9. Casting device (10) according to any one of the preceding claims, wherein, in the assembled state of the distributor (12), the distributor component (14) defines an overflow (38) which is arranged and shaped such that a flow of material across a predetermined boundary surface or interface in the inner cavity (46) causes a flow of material into the overflow (38).

10. Casting device (10) according to any one of the preceding claims, wherein at least the distributor (12) is reusable,
and/or
wherein the opening (26) and the at least one channel (28) are provided with a non-stick coating,
and/or,
wherein the distributor components (14) are formed of a material comprising aluminum.

11. Casting device (10) according to any one of the preceding claims, wherein the molded seal (44) is formed of two symmetrically formed and couplable molded seal components (45),
and/or
wherein, in the direction of a centrifugal axis of the casting device, the cassette (16) is formed of an upper (50) and lower (52) couplable cassette component (50, 52), wherein the cassette components (50, 52) are formed for receiving a respective molded seal component (45), and wherein the cassette components (50, 52) and the molded seal components (45) preferably each comprise a recess or opening (58, 62) for receiving a gas exchanger closure (40) which is concentric with the centrifugal axis of the casting device (10).

12. Method for producing a potting for a gas exchanger under the influence of a centrifugal force, comprising:
- providing a cassette (16) and inserting gas exchanger elements (40) into an inner cavity (46) of the cassette (16);
- providing a distributor (12) having at least one continuous channel (28) and mounting the distributor (12) on the cassette (16) such that an opening (26) of the distributor (12) is fluidically connected via the channel (28) to the cavity to form a casting device (10) for producing a potting for a gas exchanger; and
- feeding a potting material into the opening (26) under the influence of a centrifugal force,
wherein the attaching of the distributor (12) comprises assembling distributor components (14) to define the opening (26) of the distributor (12) and to form the channel (28) between adjacent regions of the distributor components (14),
**characterized in that**
the method is carried out by using a casting device (10) according to any one of claims 1 to 11.

13. Method according to claim 12, wherein the assembling of the distributor components (14) comprises attaching an inner seal (30) and an outer seal (32) on opposite sides of the respective channel (28) of each distributor component (14) to fluidly seal the respective channel (28) in the assembled state of the distributor (12).

14. Method according to claim 12 or 13, wherein providing the cassette (16) includes:
- providing a molded seal (44) which is formed from two couplable molded seal components (45), wherein the cassette (16) is closable and formed to receive the molded seal (44) and wherein the cassette (16) is formed of an upper and lower couplable cassette component (50; 52) in the direction of a centrifugal axis of the casting device (10);
- inserting a first molded seal component (45) into the lower cassette component (52) and placing the gas exchanger elements (40) on the first molded seal component (45);
- placing the second molded seal component (45) on the first molded seal component (45); and
- placing the upper cassette component (50) on the second molded seal component (45) so that the molded seal (44) completely encloses the inner cavity and fluidly or fluid-tightly seals the inner cavity up to the at least one channel (28).

## Revendications

1. Dispositif d'enrobage (10) pour la fabrication d'un enrobage pour un échangeur de gaz sous l'action d'une force centrifuge, comportant :
- un distributeur (12), lequel comporte une ouverture (26) et au moins un canal (28) continu et est destiné à recevoir un matériau d'enrobage fluide par l'ouverture (26) et à guider celui-ci par l'intermédiaire de l'au moins un canal (28), dans lequel le distributeur (12) comporte au moins deux composants de distributeur (14), lesquels, à l'état assemblé du distributeur (12), définissent l'ouverture (26), sont reliés les uns aux autres de manière étanche contre les fuites et forment l'au moins un canal (28) entre des zones adjacentes ; et
- une cassette (16), laquelle définit une cavité interne (46) pour la réception d'éléments d'échangeur de gaz (40), laquelle cavité interne est reliée fluidiquement à l'au moins un canal (28), **caractérisé en ce que**
le dispositif d'enrobage (10) comporte un joint d'étanchéité profilé (44), dans lequel la cassette (16) est verrouillable et conçue pour la réception du joint d'étanchéité profilé (44), dans lequel le joint d'étanchéité profilé (44) entoure complètement la cavité interne (46) et étanchéifie celle-ci contre les fluides jusqu'à l'au moins un canal (28).

2. Dispositif d'enrobage (10) selon la revendication 1, dans lequel les composants de distributeur (14), à l'état assemblé du distributeur (12), forment au moins deux canaux (28) continus.

3. Dispositif d'enrobage (10) selon la revendication 1 ou 2, dans lequel le distributeur (12) est formé de composants de distributeur (14) uniformes.

4. Dispositif d'enrobage (10) selon la revendication 3, dans lequel chaque composant de distributeur (14), à l'état assemblé du distributeur (12), est adjacent à deux composants de distributeur (14) et forme un canal (28) avec le composant de distributeur (14) adjacent,
dans lequel le distributeur (12) est formé de préférence de trois ou quatre composants de distributeur (14) uniformes.

5. Dispositif d'enrobage (10) selon l'une des revendications précédentes, dans lequel le distributeur (12) est formé de trois ou quatre composants de distributeur (14) uniformes et dans lequel chaque composant de distributeur (14), à l'état assemblé du distributeur (12), est adjacent à deux composants de distributeur (14) et forme un canal (28) avec le composant de distributeur (14) adjacent.

6. Dispositif d'enrobage (10) selon l'une des revendications précédentes, dans lequel les composants de distributeur (14) sont destinés à être reliés les uns aux autres de manière amovible, et sont destinés à un assemblage réversible du distributeur (12).

7. Dispositif d'enrobage (10) selon l'une des revendications précédentes, dans lequel chaque composant de distributeur (14) comporte un joint d'étanchéité interne (30) et un joint d'étanchéité externe (32) sur des côtés opposés du canal (28) respectif, lesquels étanchéifient fluidiquement le canal (28) respectif à l'état assemblé du distributeur (12).

8. Dispositif d'enrobage (10) selon l'une des revendications précédentes, dans lequel l'ouverture (26) définit une zone de remplissage (36) en forme d'entonnoir pour le matériau d'enrobage,
et/ou
dans lequel l'ouverture (26) s'étend jusqu'à une surface externe de la cassette (16) et relie fluidiquement celle-ci à l'environnement et dans lequel l'ouverture (26) est concentrique avec un axe centrifuge du dispositif d'enrobage (10).

9. Dispositif d'enrobage (10) selon l'une des revendications précédentes, dans lequel le composant de distributeur (14), à l'état assemblé du distributeur (12), définit un trop-plein (38), lequel est disposé et façonné de telle sorte qu'un flux de matériau sur une surface limite prédéfinie dans la cavité interne (46) provoque un flux de matériau dans le trop-plein (38).

10. Dispositif d'enrobage (10) selon l'une des revendications précédentes, dans lequel au moins le distributeur (12) est réutilisable,
et/ou
dans lequel l'ouverture (26) et l'au moins un canal (28) sont pourvus d'un revêtement antiadhésif,
et/ou
dans lequel les composants de distributeur (14) sont constitués d'un matériau comportant de l'aluminium.

11. Dispositif d'enrobage (10) selon l'une des revendications précédentes, dans lequel le joint d'étanchéité profilé (44) est constitué de deux composants de joint d'étanchéité profilé (45) symétriques et pouvant être accouplés,
et/ou
dans lequel la cassette (16) est constituée de parties structurales de cassette (50, 52), une partie structurale de cassette supérieure (50) et une partie structurale de cassette inférieure (52), pouvant être accouplées dans la direction d'un axe centrifuge du dispositif d'enrobage, dans lequel les parties structurales de cassette (50, 52) sont conçues pour la réception d'un composant de joint d'étanchéité profilé (45) respectif et dans lequel les parties structurales de cassette (50, 52) et les composants de joint d'étanchéité profilé (45) comportent de préférence respectivement un évidement ou une ouverture (58, 62) pour la réception d'un enrobage d'échangeur de gaz (40), lequel évidement ou laquelle ouverture est concentrique avec l'axe centrifuge du dispositif d'enrobage (10).

12. Procédé pour la fabrication d'un enrobage pour un échangeur de gaz sous l'action d'une force centrifuge, comportant :
- la fourniture d'une cassette (16) et l'utilisation d'éléments d'échangeur de gaz (40) dans une cavité interne (46) de la cassette (16) ;
- la fourniture d'un distributeur (12) avec au moins un canal (28) continu et la fixation du distributeur (12) sur la cassette (16) de sorte qu'une ouverture (26) du distributeur (12) soit reliée fluidiquement par l'intermédiaire du canal (28) à la cavité, afin de former un dispositif d'enrobage (10) pour la fabrication d'un enrobage pour un échangeur de gaz ; et
- l'introduction d'un matériau d'enrobage dans l'ouverture (26) sous l'action d'une force centrifuge,
dans lequel la fixation du distributeur (12) comporte l'assemblage de composants de distributeur (14) afin de définir l'ouverture (26) du distributeur (12) et de former le canal (28) entre des zones adjacentes des composants de distributeur (14),
**caractérisé en ce que**
le procédé est exécuté au moyen d'un dispositif d'enrobage (10) selon l'une des revendications 1 à 11.

13. Procédé selon la revendication 12, dans lequel l'assemblage des composants de distributeur (14) comporte l'application d'un joint d'étanchéité interne (30) et d'un joint d'étanchéité externe (32) sur des côtés opposés du canal (28) respectif au niveau de chaque composant de distributeur (14), afin d'étanchéifier fluidiquement le canal (28) respectif à l'état assemblé du distributeur (12).

14. Procédé selon la revendication 12 ou 13, dans lequel la fourniture de la cassette (16) comporte :
- la fourniture d'un joint d'étanchéité profilé (44), lequel est constitué de deux composants de joint d'étanchéité profilé (45) pouvant être accouplés, dans lequel la cassette (16) est verrouillable et conçue pour la réception du joint d'étanchéité profilé (44) et dans lequel la cassette (16) est constituée d'une partie structurale de cassette supérieure (50) et d'une partie structurale de cassette inférieure (52) pouvant être accouplées dans la direction d'un axe centrifuge du dispositif d'enrobage (10) ;
- l'introduction d'un premier composant de joint d'étanchéité profilé (45) dans la partie structurale de cassette inférieure (52) et la mise en place des éléments d'échangeur de gaz (40) sur le premier composant de joint d'étanchéité profilé (45) ;
- la mise en place du deuxième composant de joint d'étanchéité profilé (45) sur le premier composant de joint d'étanchéité profilé (45) ; et
- la mise en place de la partie structurale de cassette supérieure (50) sur le deuxième composant de joint d'étanchéité profilé (45) de sorte que le joint d'étanchéité profilé (44) entoure complètement la cavité interne et étanchéifie celle-ci contre les fluides jusqu'à l'au moins un canal (28).
